# EUROPEAN PATENT SPECIFICATION

(11) **EP 2 163 545 B1**
(45) Date of publication and mention of the grant of the patent: **29.10.2014**
(21) Application number: 08765433.1
(22) Date of filing: 11.06.2008
(51) Int. Cl.: C07D 233/58, C07F 9/6568

(54) **IONIC LIQUID AND METHOD FOR PRODUCING THE SAME**
IONISCHE FLÜSSIGKEIT UND VERFAHREN ZU DEREN HERSTELLUNG
LIQUIDE IONIQUE ET SON PROCÉDÉ DE FABRICATION

(30) Priority: 12.06.2007 JP 2007155769
(43) Date of publication of application: 17.03.2010
(73) Proprietor: Nippoh Chemicals Co., Ltd, Tokyo 103-0023 (JP)
(72) Inventor: MITSUI, Hitoshi, Chiba 298-0104 (JP); SONOBE, Kenji, Tokyo 103-0023 (JP); RYU, Ilhyong, Osaka 599-8531 (JP); FUKUYAMA, Takahide, Osaka 599-8531 (JP)
(74) Representative: Métay, Émeline
(86) International application number: PCT/JP2008/060654
(87) International publication number: WO 2008/153045

(56) References cited:
- EP-A1- 1 498 409
- WO-A1-03/091198
- POTEWAR ET AL: "Efficient and rapid synthesis of 1-substituted-1H-1,2,3,4-tetrazoles in the acidic ionic liquid 1-n-butylimidazolium tetrafluoroborate", TETRAHEDRON LETTERS, ELSEVIER, AMSTERDAM, NL, vol. 48, no. 10, 8 February 2007 (2007-02-08), pages 1721-1724, XP005879163, ISSN: 0040-4039, DOI: DOI:10.1016/J.TETLET.2007.01.050
- ONO H.: 'Ion Ekitai II -Kyoiteki na Shinpo to Tasai na Kinmirai-, 1st. print', 30 March 2006, CMC PUBLISHING CO., LTD. pages 46 - 47, XP008118310

## Description

### Technical Field

The present invention relates to a method for producing an ionic liquid.

### Background Art

Ionic liquids (which are also referred to as ion liquids, ionic fluids, or ambient temperature molten salts) are molten salts constituted by combining a cation and an anion. One of characteristics of ionic liquids is to be in a liquid state at a wide range of temperatures from -50°C to 400°C. Not a few of ionic liquids are known to be liquid at room temperatures (25°C).

Apart from being liquid at such a wide temperature range, ionic liquids have such characteristics as involatility due to their very low vapor pressure, excellent heat stability, excellent electrochemical stability, low viscosity, and high ionic conductivity. By utilizing these characteristics, ionic liquids are employed in electric devices such as electrolyte solution compositions in which an electrolyte solution or electrolyte is dissolved, dye-sensitized solar cells, fuel cells, lithium-ion secondary battery, electrical double layer capacitors, etc.

Moreover, ionic liquids are highly polarized due to their aprotic ion structure, and therefore show excellent dissolving power for organic and inorganic compounds. By utilizing this characteristic, many attempts have been made to use ionic liquids as reaction solvents for organic synthesis. In case where an ionic liquid is used as a reaction solvent, a solute is solvated with ions. Therefore, a reaction takes place under an environment utterly different from a case where as the reaction solvent is water or an organic solvent. Moreover, some ionic liquids are difficult to dissolve in water and lowly-polarized organic solvents. By using such ionic liquids, multiphase environment can be provided. Furthermore, ionic liquids are almost involatile, and therefore excellent in terms of safety. Consequently, ionic liquids are highly expected as reaction solvents.

Typical examples of a method for producing an ionic liquid encompass anion-exchange method, acid-ester method, and neutralization method. The anion-exchange method is more popular than the acid-ester method and the neutralization method which are capable for producing a limited kinds of ionic liquids. The anion-exchange method is a synthesis method utilizing an anion-exchange reaction. In the anion-exchange method, a halide containing a cation to constitute a target ionic liquid is reacted with a salt containing an anion constitute the target ionic liquid, so as to synthesize the target ionic liquid (see Non-Patent Literature for example).

As described above, an ionic liquid is a molten salt formed by combining a cation and an anion. It is preferable that the ionic liquid is constituted of the cation and the anion purely. In reality, however, the ionic liquid contains a trace of impurity such as water, for example. Contamination or generation of the impurity mainly occurs during the synthesis process of the ionic liquid. The impurity largely affects the ionic liquid thus produced. For example, in case the ionic liquid contains water, the water therein affects the properties of the ionic liquid. For example, the water therein reduces the viscosity of the ionic liquid. Moreover, in case where the ionic liquid is used as a reaction solvent, the water therein adversely affects reactivity. For example, the water therein becomes coordinated with a catalyst metal, thereby affecting a reaction rate. In case where the ionic liquid is used as an electrolyte solution, the water therein brings about by-production, thereby being a cause of product quality deterioration.

Moisture absorption from atmosphere can be another cause of the contamination with water. The moisture absorption from atmosphere is a significant problem for hydrophilic ionic liquids, especially. However, even hydrophobic ionic liquids absorb 1 wt% or more moisture from atmosphere. Therefore, regardless of whether the ionic liquid is hydrophilic or hydrophobic, removal of water from the ionic liquid should be performed.

The removal of water from the ionic liquid is carried out by using well-known water removal methods such as vacuum drying under high vacuum and high temperature condition, use of a molecular sieve, and absorptive dehydration using silica or alumina. Moreover, Patent Literature 1 discloses a method of removing water from an ionic liquid by breaking down the water into hydrogen and oxygen. As other measures apart from the water removal, the synthesis reaction of the ionic liquid is optionally carried out in a dry room in which humidity can be controlled by ppm order.

### Citation List

Non-Patent Literature 1
   "Ionic Liquid", edited by Hiroyuki ONO, CMC publishing Co. Ltd. (2003).
Patent Literature 1
   International Publication No. 2003/091198 pamphlet (published on January 19, 2003)

### Summary of Invention

However, the water removal by using the well-known method has various problems. For example, the water removal by vacuum drying requires a time period of 24 hours or longer in order to dry the liquid crystal to such water content that does not affect the property of the ionic liquid thus produced. Thus, the water removal takes a long time in case where the vacuum drying is adopted. Especially, for hydrophilic ionic liquids, which are very moisture absorptive, the water removal by vacuum drying would not remove the moisture sufficiently even after the vacuum drying is carried out for 24 hours or longer. Therefore, the production of a hydrophilic ionic liquid requires very tight control for usage of water as the reaction solvent and very strict moisture management during the synthetic reaction. This complicates the production method of the ionic liquid and makes it very difficult to produce the ionic liquid on industrial basis.

Moreover, in case where the water removal is carried out by using the adsorptive dehydration, it is necessary to dry an adsorbent agent. This increases the number of steps in the production of the ionic liquid, thereby complicating the production method of the ionic liquid. Furthermore, if the ionic liquid to be produced has a high viscosity, the adsorbent agent cannot be used. Further, the use of the dry room requires very high installation cost. Further the use of the dry room does not allow use of water during the synthesis. This limits the kinds of ionic liquid to be produced. Meanwhile, the use of water electrolysis requires a very large amount of electric energy in order to produce the ionic liquid on industrial basis. This increases production cost of the ionic liquid.

The present invention is made in view of these problems. Mainly, an object of the present invention is to provide a method of economically, easily, and quickly producing an ionic liquid of any kind with low water content.

As a result of diligent studies, the inventors of the present invention found that it is easier to remove an organic solvent from an ionic liquid than to remove water from the ionic liquid. The inventors of the present invention also demonstrated that water in a molten salt solution obtained by producing a molten salt can be converted to an organic solvent by adding a compound that is hydrolysable with water thereby to produce the organic solvent. The present invention is based on these findings.

A method according to the present invention is a method for producing an ionic liquid from a molten salt solution containing a molten salt constituted by combining the cation portion and the anion portion, the ionic liquid containing the molten salt as a main component, the method comprising:
removing water from the molten salt solution, the step of removing the water including adding a compound in the molten salt solution, the compound being represented by General Formula (1): 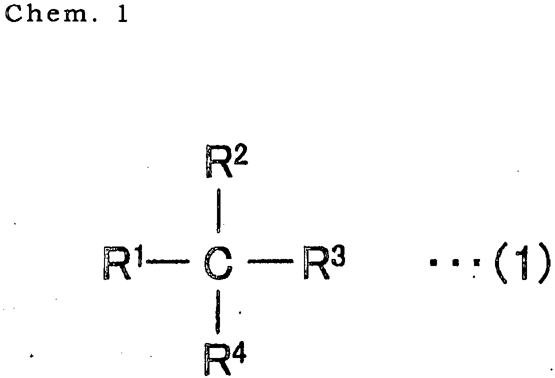 where at least two of R¹ to R⁴ are a C1 to C8 alkoxy group(s), the rest of R¹ to R⁴ are a hydrogen atom(s) or a C1 to C8 alkyl group(s)
   and
   purifying a mixture obtained by the step of removing the water, the step of purifying removing at least one of a compound represented by General Formula (2) and an alcohol represented by General Formula (3),
   General Formula (2) being as follows: 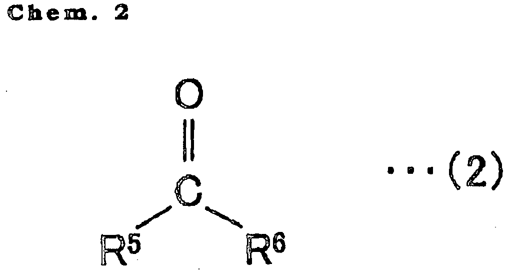 where R⁵ and R⁶ are a hydrogen atom(s), a C1 to C8 alkyl group(s), or a C1 to C8 alkoxy group(s), and
   General Formula (3) being as follows: Chem. 3

   R⁷-OH (3)

   where R⁷ is a C1 to C8 alkyl group.

The method according to the present invention preferably further comprises: synthesizing the molten salt, wherein: in the step of removing the water, the compound is added to the molten salt solution obtained in the step of synthesizing the molten salt.

With these arrangement, the compound represented by General Formula (1) is hydrolyzed with water in the molten salt solution, thereby generating a ketone or an ester, and alcohols. That is, the water difficult to remove from the molten salt solution can be converted into an organic solvent that is easy to remove from the molten salt solution.

This significantly shortens a time required to remove the water. Further, this makes it possible to remove the water without using an adsorbent agent such as a molecular sieve or silica, thereby eliminating a cumbersome step of drying the adsorbent agent. Moreover, it is not necessary to install an expensive equipment in addition. Thus, it is possible to reduce the cost of production of the ionic liquid.

Because the hydrolysis reaction of the compound represented by General Formula (1) is a reaction between this compound and water, these arrangement can remove the water from the molten salt solution, regardless of whether the molten salt is hydrophilic or hydrophobic.

In this Description etc., the "molten salt solution" is a solution obtained by generating a molten salt constituted by combining a cation portion and an anion portion, and has a water content greater than 200ppm and contains the molten salt as its main component.

The method according to the present invention is preferably arranged such that: the compound represented by General Formula (1) is an acetal or an orthoester compound.

An acetal and an orthoester are stable compounds that are easy to handle and very rarely cause a side reaction. Thus, the use of an acetal or an orthoester makes it easier to carry out the hydrolysis reaction.

The method according to the present invention is preferably arranged such that: the acetal or the orthoester compound is one selected from the group consisting of dimethoxymethane, diethoxymethane, 1,1-dimethoxyethane, 1,1-diethoxyethane, 1,1-dimethoxypropane, 1,1-diethoxypropane, 2,2-dimethoxypropane, 2,2-diethoxypropane, 1,1-dimethoxybutane, 1,1-diethoxybutane, 2,2-dimethoxybutane, 2,2-diethoxybutane, orthomethyl formate, orthoethyl formate, orthomethyl acetate, and orthoethyl acetate.

The method according to the present invention is preferably further comprises: purifying a mixture obtained by the step of removing the water, the step of purifying removing at least one of a compound represented by General Formula (2) and an alcohol represented by General Formula (3), General Formula (2) and General Formula (3) being as follows: 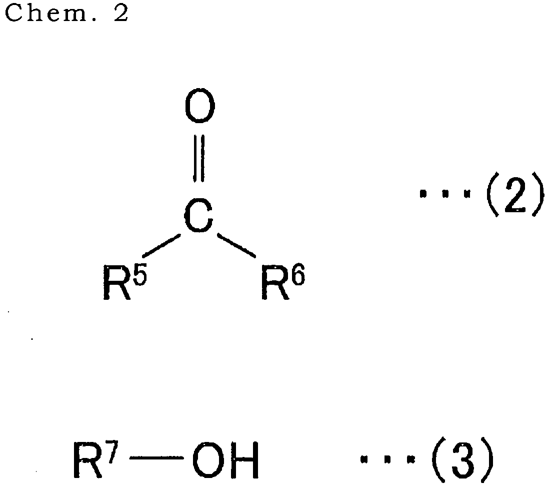 where R⁵ and R⁶ are a hydrogen atom(s), a C1 to C8 alkyl group(s), or a C1 to C8 alkoxy group(s), and where R⁷ is a C1 to C8 alkyl group.

By including this step, it is possible to obtain an ionic liquid with high purity in which impurity content is very low.

The method according to the present invention is preferably arranged such that: the compound represented by General Formula (2) is a ketone, an aldehyde, or an ester.

A ketone, an aldehyde, and an ester are stable compounds that will not be broken down in the ionic liquid thereby forming another by-product therein. Thus, by removing in the form of the ketone, the aldehyde, or the ester, it is possible to obtain an ionic liquid with high purity.

The method according to the present invention is preferably arranged such that: the compound represented by General Formula (2) and the alcohol represented by General Formula (3) have boiling points of 200°C or lower.

With this arrangement, the compound represented by General Formula (2) and the alcohols represented by General Formula (3), which are generated by the hydrolysis, can be removed easily from the ionic liquid.

The method according to the present invention is preferably arranged such that: the cation portion is a quaternary ammonium cation or a cation having a heterocyclic skeleton having a nitrogen atom,
the quaternary ammonium cation being represented by General Formula (4): 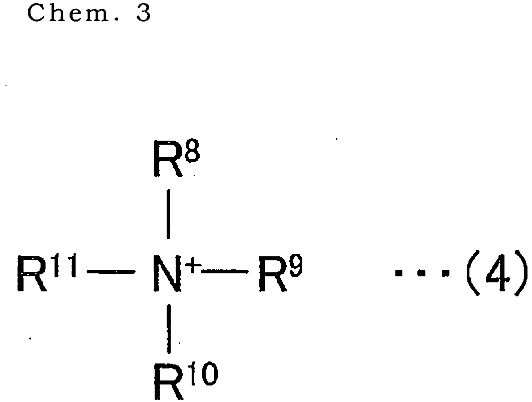 where R⁸ to R¹¹ are a saturated alkyl group(s), an unsaturated alkyl group(s), a cycloalkyl group(s), a heterocyclic group(s), an aryl group(s), or an alkoxyalkyl group(s).

The method according to the present invention is preferably arranged such that: the cation having the heterocyclic skeleton is an imidazolium cation being represented by G-eneral Formula (5),a pyridinium cation being represented by General Formula (6), or the triazine derivative cation being represented by General Formula (7): 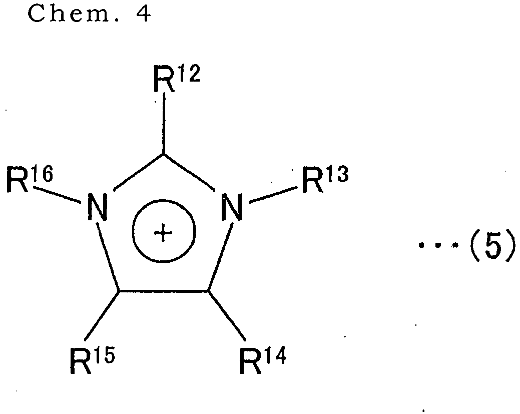 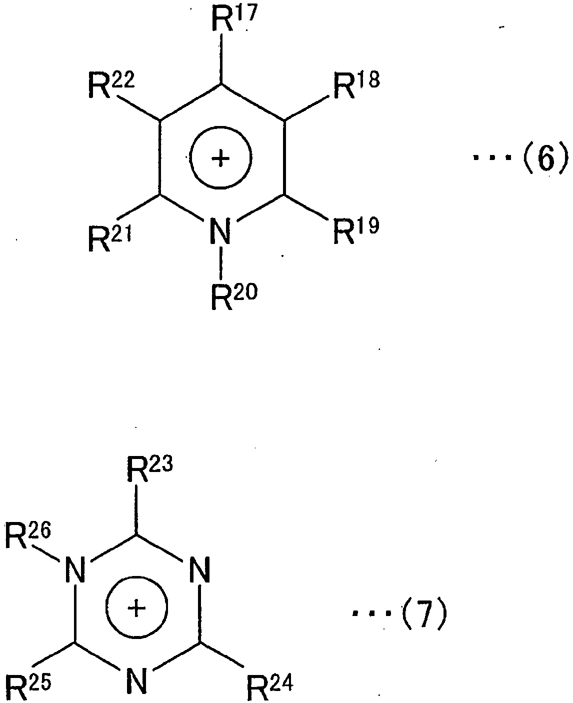 where R¹² to R²⁶ are a hydrogen atom(s), a saturated alkyl group(s), an unsaturated alkyl group(s), a cycloalkyl group(s), an aryl group(s), or alkoxyalkyl group(s),

The method according to the present invention is preferably arranged such that: the cation portion is a quaternary phosphonium cation, or a cation having a heterocyclic skeleton having a phosphorous atom,
the quaternary phosphonium cation being represented by General Formula (8): 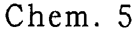 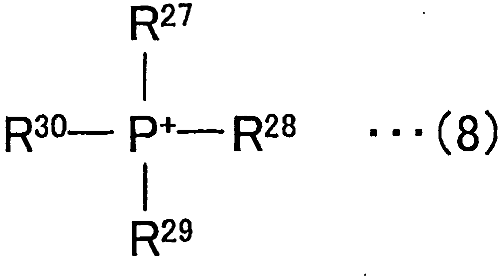 where R²⁷ to R³⁰ are a saturated alkyl group(s), an unsaturated alkyl group(s), a cycloalkyl group(s), a hetrocyclic group(s), an aryl group(s), a vinyl group(s), or an alkoxyalkyl group(s).

The method according to the present invention is preferably arranged such that: the anion portion is one selected from the group consisting of: a halide ion, triflate anion, tetrafluoroborate anion, hexafluorophosphate anion, nitrate ion, sulfate ion, N(CF₃SO₂)₂-, N(CN)₂-, C(CN)₃-, CH₃OSO₃-, (C₂H₅)₂PO₄-, N(C₂F₅SO₂)₂-, N(CF₃CO)₂-, N(CF₃SO₂)(CF₃CO)-, N(FSO₂)₂-, and CF₃COO-.

With these arrangement, the influence from the water in the ionic liquid can be reduced as much as possible. For example, in case the ionic liquid is used as a reaction solvent, the influence of reaction rate from the water coordinating with a catalyst metal can be reduced as much as possible. In case where the ionic liquid is used as an electrolyte, this prevents produce quality deterioration caused by generation of a by-product.

The ionic liquid preferably comprises: the compound represented by General Formula (2) by 200ppm or less; and the alcohol represented by General Formula (3) by 200ppm or less.

With this arrangement, the ionic liquid can be very low in impurity content, thereby achieving a high purity. For example, such an ionic liquid is more applicable to reaction solvents or electric devices.

For a fuller understanding of the nature and advantages of the invention, reference should be made to the ensuing detailed description taken in conjunction with the accompanying drawings.

### Description of Embodiments

### [Embodiment 1]

One embodiment of a method according to the present invention for producing an ionic liquid is described below.

Here, details of the method for producing the ionic liquid are described first. Then, materials suitable for the method according to the present invention for producing the ionic liquid are described.

### (Properties of Ionic Liquid)

The ionic liquid is a composition, which is also referred to as ion liquid, ionic fluid, or ambient temperature molten salt. More specifically, the ionic liquid is a composition whose main component is a molten salt constituted by bonding a cation portion and an anion portion. In this Description etc., the term "main component" is a component that takes 50% by mass or more of the whole composition. It is preferable that the main component takes 90% by mass or more of the composition, and it is more preferable that the main component takes 100% by mass or more of the composition.

In the present Description etc., what is meant by the term "molten salt" is a salt that is constituted by combining a cation portion and an anion portion, and that is in a liquid state at a temperature in a range of -50°C to 400°C. It is preferable that the salt is in the liquid state around room temperatures (about 25°C).

The ionic liquid, and its cation portion and the anion portion preferably applicable to the present invention will be described later.

### (Method for producing Ionic Liquid)

The method according to the present invention for producing the ionic liquid includes the water removing step of removing water from a molten salt solution containing the molten salt. Moreover, the method according to the present invention for producing the ionic liquid includes the synthesis step of synthesizing the molten salt solution and the purifying step of purifying a mixture obtained in the water removing step. Theses steps are described below.

In this Description etc., the term "ionic liquid" indicates a molten salt having a water content of 200ppm or less. The term "molten salt solution" indicates a solution that is obtained by producing the molten salt, has a water content of greater than 200ppm, and contain the molten salt as its main component.

### (Synthesis step)

The synthesis step is a step for synthesizing the molten salt by combining the cation portion and the anion portion. The synthesis step may be carried out by employing a conventionally known synthesizing method. Specific examples of the synthesizing method of the molten salt encompass an anion-exchange method, acid-ester method, neutralization method, etc. For greater varieties of the molten salt to be produced, and applicability to industrial production, it is preferable to use the anion-exchange method.

The present embodiment describes an example where the molten salt is synthesized by using the anion-exchange method. The anion-exchange method is a method of synthesizing a molten salt by reacting a salt containing a cation to constitute a target ionic liquid, with a salt containing an anion constitute the target ionic liquid.

An anion-exchange reaction of the anion-exchange method also produces a by-product salt. Therefore, in case the synthesizing method is carried out by using the anion-exchange method, the synthesizing method should includes the by-product salt removing step for removing the by-product salt.

The by-product salt removing step may be carried out by using a method conventionally known. More specifically, to produce a hydrophobic ionic liquid, phase separation using water can be adopted, which can easily remove the by-product salt. To produce a hydrophilic ionic liquid, such phase separation using water cannot be adopted. Therefore, the removal of the by-product salt is generally carried out by column chromatography or filtration. More specifically, the column chromatography may use a column filled with alumina, for example. The filtration may be carried out with a silver salt such as silver nitrate or the like as a starting material. This converts the by-product salt into a silver salt insoluble in the molten salt solution.

Moreover, the removal of the by-product salt may be carried out by precipitating the by-product salt from the molten salt solution in which the by-product salt is dissolved, and then filtering out the by-product salt thus precipitated. More specifically, a solvent used in the synthesis reaction is removed after the anion-exchange reaction, so as to prepare a slurry containing the by-product salt and the molten salt solution. Then, a solvent is added to the slurry, so as to precipitate the by-product salt.

The solvent for precipitating the by-product salt is preferably a solvent to which solubility of the by-product salt is low, that is a poor solvent for the by-product salt. More specifically, it is preferable that the solvent for precipitating the by-product salt be a ketone-type solvent or a halogen-type solvent. It is more preferable that the solvent for precipitating the by-product salt be acetone or chloroform.

The use of a poor solvent for the by-product salt in order to precipitate the by-product salt makes the removal of the by-product salt easier and more economical. Further, the removal of the by-product salt by using the poor solvent for the by-product salt is applicable to the production of the hydrophilic ionic liquid and the production of the hydrophobic ionic liquid, either. This eliminates the need of using water in the salt removal in the production of the hydrophobic ionic liquid. Therefore, this can reduce an amount of water to remove in the water removing step described below.

Moreover, for the use of such a poor solvent, it is preferable that the anion constituting the ionic liquid is added as a salt with a cation that is an alkali metal cation or an alkali earth metal cation, even though the present invention is not particularly limited in terms of kinds of this salt, unlike the filtration using a silver salt such as silver nitrate. In other words, it is preferable to convert the by-product salt to an alkali metal salt or an alkali earth metal salt. This makes it easier to precipitate the by-product salt in the poor salt for the by-product salt.

### (Water Removing Step)

The water removing step removes moisture contained in the molten salt solution containing the molten salt. The water removing step may remove the water from the molten salt solution thus prepared by synthesizing the molten salt in the synthesis step. As described above, high water content in the ionic liquid affects the properties of the ionic liquid. When the ionic liquid is used as a reaction solvent, for example the water is coordinated with a catalytic metal, thereby affecting the reaction rate. In this way, the high water content adversely affects reactivity. When the ionic liquid is used as an electrolyte, the high water content results in production of a by-product, thereby being a cause of product quality deterioration.

The water contained in the molten salt solution comes from water used as the solvent in the synthesis step of the molten salt, and moisture absorption from atmosphere. Because the main component of the ionic liquid is the molten salt constituted by combining the cation portion and the anion portion, the properties of the ionic liquid allows about 1 wt% moisture absorption even for the hydrophobic ionic solution. Therefore, it is necessary to remove the water from the molten salt solution by the water removing step in the production of the hydrophobic ionic solution as well as in the production of the hydrophilic ionic solution.

The water removing step according to the present invention reduces the water content in the molten salt solution to 200ppm or less.

More specifically, the water removing step in the method according to the present invention for producing the ionic liquid includes adding a compound in the molten salt solution, the compound being represented by General Formula (1): 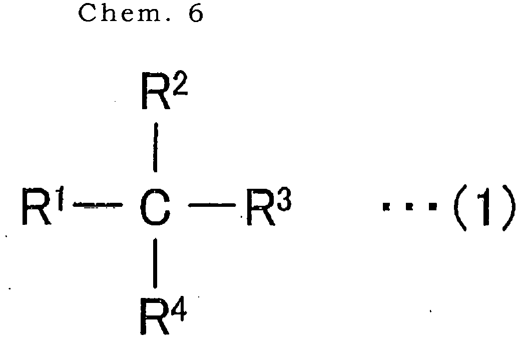 where at least two of R¹ to R⁴ are a C1 to C8 alkoxy group(s), the rest of R¹ to R⁴ are a hydrogen atom(s) or a C1 to C8 alkyl group(s).

Moreover, in this Description etc., the hydrogen atom encompass deuterium and tritium.

When the compound represented by General Formula (1) is added into the molten salt solution, the compound is hydrolyzed with water in the molten salt solution, thereby generating ketone or ester, and alcohol. That is, by the addition of the compound represented by General Formula (1), the difficult-to-remove water in the molten salt solution is converted to an easy-to-remove organic solvent.

By this, the water removal can be performed in a much shorter time. Further, the water removal can be performed without the need of a molecular sieve or an adsorptive agent such as silica. Thus, this eliminates the need of such a cumbersome step of drying the adsorptive agent. Moreover, the water removing step according to the present invention does not require expensive facility additionally, thereby avoiding cost increase for the ionic liquid production.

Moreover, the "hydrolysis" in this Description etc. is caused by attaching the primary carbon atom in General Formula (1) with oxygen atom of the water, and thereby breaking off any one of the at least two alkoxy groups in General Formula (1). More specifically, this hydrolysis requires the primary carbon atom of General Formula (1) to be poor in electrons. Therefore, it is necessary that the primary carbon atom of General Formula (1) be bonded with at least two oxygen atoms, which are great in electron attraction.

The compound represented by General Formula (1) is only required that at least two of R¹ to R⁴ are a C1 to C8 alkoxy group(s). However, for the sake of better stability the compound, it is preferable that the compound be an acetal wherein two of R¹ to R⁴ are a C1 to C8 alkoxy group(s), or that the compound be an orthoester compound wherein three of R¹ to R⁴ are a C1 to C8 alkoxy group(s). In this description etc., the term "orthoester compound" is a collective term of 1,1,1-trialkoxide compounds.

In case the compound represented by General Formula (1) is the orthoester compound, the orthoester compound reacts as illustrated in the following reaction formula (1), and thereby produce one ester (an orthoester derivative) and two molecules of alcohols. 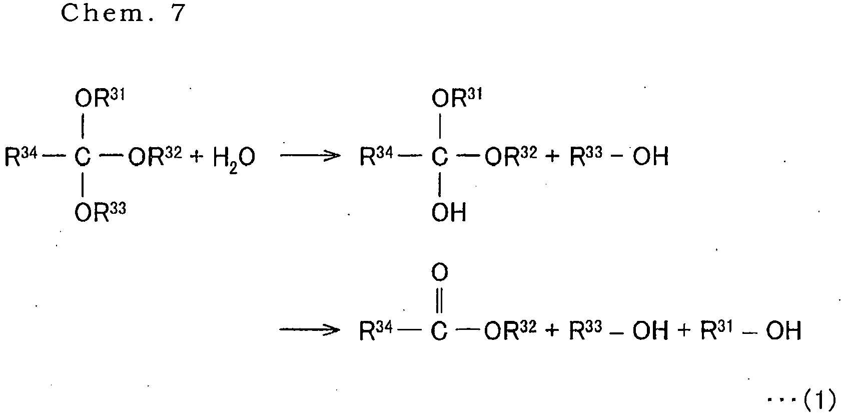 where R³¹ to R³³ are a C1 to C8 alkyl group(s), and R³⁴ is a hydrogen atom or a C1 to C8 alkyl group.

Moreover, in case where the compound represented by General Formula (1) is the acetal, the acetal reacts as illustrated in the following Reaction Formula (2), thereby producing one molecule of a ketone or aldehyde, which is an acetal derivate, and two molecules of alcohols. 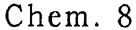 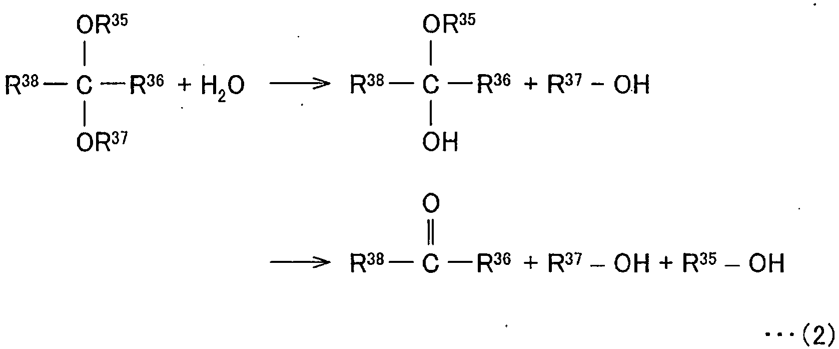 where R³⁵ and R³⁷ are a C1 to C8 alkyl group(s) and R³⁶ and R³⁸ are a hydrogen atom(s) or a C1 to C8 alkyl group(s).

Here, the C1 to C8 alkoxy group(s) may be straight or branched, provided that its carbon number is in a range of 1 to 8. As long as the carbon number of the alkoxy group is within the range, the alcohols produced by the hydrolysis have boiling points lower than that of the ionic liquid. In the purifying step described below, the alcohols with such boiling points can be easily removed.

Moreover, in general, an alcohol is lower in boiling point as its carbon chain is shorter. Thus, it is preferable that the alkoxy group have a smaller carbon number. It is more preferable that the alkoxy group have a carbon number in a range of 1 to 4. It is further preferable that the alkoxy group have a carbon number of 1 or 2. More specifically, the alkoxy group is preferably methoxy group, ethoxy group, propoxy group, or n-butoxy group. It is more preferable that the alkoxy group be methoxy group or ethoxy group.

The rest of the substituents R¹ to R⁴, other than these being the C1 to C8 alkoxy group(s), are a hydrogen atom(s) or the C1 to C8 alkyl group(s). The C1 to C8 alkyl group(s) may be straight or branched, provided that its carbon number is in a range of 1 to 8. As long as the carbon number is in this range, the ester or ketone produced from the hydrolysis are lower in boiling point than the ionic liquid. Thus, the ester or ketone can be easily removed from the ionic liquid in the purifying step described later. If any one of the rest of the substituents R¹ to R⁴ is a hydrogen atom, the hydrolysis will produce an aldehyde.

Moreover, esters, ketones, and aldehydes are generally lower in boiling point as their carbon chains are shorter. Thus, it is preferable that the alkyl group has a smaller carbon number. It is more preferable that the alkyl group has a carbon number in a range of 1 to 4. It is further preferable that the alkyl group has a carbon number of 1 or 2. More specifically, the alkyl group is preferably methyl group, ethyl group, propyl group, isopropyl group, or butyl group. It is more preferable that the alkyl group be methyl group or ethyl group.

As described above, the compound presented by General Formula (1) is preferably one selected from the group consisting of dimethoxymethane, diethoxymethane, 11-dimethoxyethane, 1,1-diethoxyethane, 1,1-dimethoxypropane, 1,1-diethoxypropane, 2,2-dimethoxypropane, 2,2-diethoxypropane, 1,1-dimethoxybutane, 1,1-diethoxybutane, 2,2-dimethoxybutane, 2,2-diethoxybutane. Among the orthoester compounds, orthomethyl formate, orthoethyl formate, orthomethyl acetate, and orthoethyl acetate are more preferable.

Note that the compound represented by General Formula (1) may be of one type, or of two types in combination.

Boiling points of the alcohols produced by the hydrolysis of the compound represented by General Formula (1) are preferably 200°C or less, and more preferably 100°C or less. More specifically, it is preferable that the alcohols be methanol, ethanol, 1-propanol, isopropanol, n-butanol, i-butanol, s-butanol, or t-butanol. It is preferable that the alcohols be methanol or ethanol. The alcohol boiling points within the range makes it easier to remove the alcohols in the purifying step described below.

That is, the alkoxy groups among R¹ to R⁴ in General Formula (1) have a carbon number in a range of, preferably 1 to 4, and more preferably of 1 or 2.

Moreover, the ester, ketone, or aldehyde produced from the hydrolysis of the compound represented by General Formula (1) has a boiling point of preferably 200°C or less, and more preferably 100°C or less. More specifically, it is preferable that the ester, ketone, or aldehyde produced from the hydrolysis be formaldehyde, acetaldehyde, propionaldehyde, n-butylaldehyde, acetone, 2-butanoe, 2-pentanone, 3-pentaone, methyl formate, ethyl formate, methyl acetate, ethyl acetate, methyl propionate, or ethyl propionate. The ester, ketone, or aldehyde produced from the hydrolysis with a boiling point within the range can be easily removed by the purifying step described below.

That is, the substituents other than alkoxy group among R¹ to R⁴ in General Formula (1) are preferably a hydrogen atom(s) or a C1 to C4 alkyl group(s), and more preferably a hydrogen atom(s) or a C1 to C2 alkyl group(s).

The water removing step reduces the water content of the molten salt solution to 200ppm or less, more preferably to 100ppm or lees, further preferably to 10ppm or less, and most preferably to 0ppm.

By reducing the water content of the molten salt solution to the ranges above by the water removing step, influence from the water contained in the ionic liquid to the properties of the ionic liquid can be reduced as much as possible. In case where the ionic liquid is used as a reaction solvent, for example, it can be achieved by reducing the influence of the water to the properties of the ionic liquid that the influence of the water to the reaction rate by coordinating with the catalyst metal is reduced. In case where the ionic liquid is used as an electrolyte, for example, it can be achieved by reducing the influence of the water to the properties of the ionic liquid that product quality deterioration due to the production of a by-product can be reduced.

### (Purifying Step)

By the purifying step, the ester or ketone, and the alcohol generated in the water removing step is removed from the mixture obtained by the water removing step.

The purifying step can be carried out by any technique that can remove the organic solvent from the mixture. More specifically, it is preferable to adopt factional distillation to separate the organic solvent from the mixture thereby purifying the mixture, because the ionic solvent is involatile, and its boiling point is much higher than those of the ester or ketone, and the alcohol thus produced. Mover, in order to shorten the time of the purifying step, it is more preferable that the factional distillation is carried out under reduced pressure by using a vacuum pump or the like.

In the evaporation under reduced pressure by using a vacuum pump or the like, the reduced pressure is preferably 20mmHg or less, more preferably 10mmHg or less, and further preferably 2mmHg or less. Moreover, it is preferable that the evaporation under reduced pressure is carried out for 1 hour or longer.

By performing the evaporation under such pressure in the range for such a time period in the range, it is possible to completely remove the ester or ketone, and the alcohols from the mixture. By this, it is possible to produce an ionic liquid of high purity.

Moreover, the purifying step reduces concentrations of the ester or ketone, and the alcohols preferably to 200ppm or less, more preferably to 100ppm or less, and most preferable to 0ppm.

By reducing the concentration of the ester or ketone, and the alcohols in the ionic liquid to theses ranges by the purifying step, it is possible to produce an ionic liquid of higher purity in which impurity content is very small.

### (Ionic Liquid)

Next, the ionic liquid suitably producible by the present invention is described below.

The ionic liquid producible by the present invention is not particularly limited. By the present invention, both hydrophilic ionic liquids and hydrophobic ionic liquids can be produced. That is, the cation portion and anion portion constituting the ionic liquid are not particularly limited as long as they can be combined to form an molten salt. Cation portions and anion portions suitable for the present invention are described below for more details.

In this Description, the term "hydrophobic" means that a compound is low in affinity with water, that is, poorly soluble in water, but does not mean that the compound is completely insoluble in water. Moreover, the term "hydrophilic" means that a compound is high in affinity with water, that is, the compound is easy to dissolve in water.

### (Cation Portion)

The cation portion of the ionic liquid producible by the production method of the present invention may have a chain structure or a cyclic structure.

Specific examples of the cation portion with a chain structure encompass: a quaternary ammonium cation represented by General Formula (4), and a quaternary phosphonium cation represented by General Formula (8). 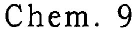 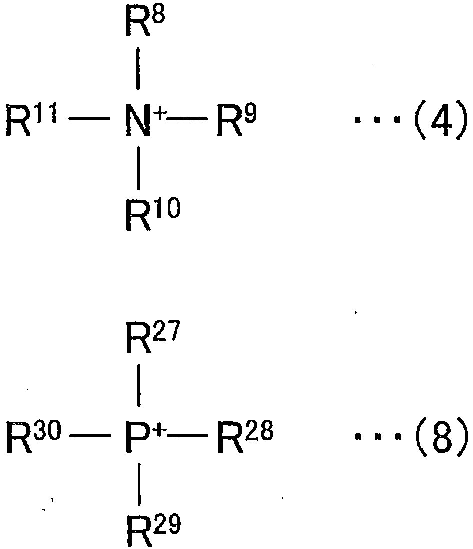 where R⁸ to R¹¹ and R²⁷ to R³⁰ are a saturated alkyl group(s), an unsaturated alkyl group(s), a cycloalkyl group(s), a heterocyclic group(s), an aryl group(s), or an alkoxyalkyl group(s).

Among these, trimethylpropyl ammonium cation, triphenyl octyl phosphonium cation, tri-t-butyl octyl phosphonium cation are more preferable.

Specific examples of the cation portion with a cyclic structure encompass a cation having a hetrocyclic skeleton having a nitrogen atom or a phosphorus atom. In the cation having such a hetrocyclic skeleton having a nitrogen atom or a phosphorus atom, the hetrocyclic skeleton is not particularly limited as to how many members it has. However, it is preferable that the hetrocyclic skeleton be 5-membered or 6-membered. The heterocyclic skeleton may be monocyclic or polycyclic. The heterocyclic skeleton may have an oxygen atom or sulfur atom in addition to the nitrogen atom or the phosphorus atom.

The specific examples of the cation having such a hetrocyclic skeleton having a nitrogen atom or a phosphorus atom encompass compounds having the following skeletons: 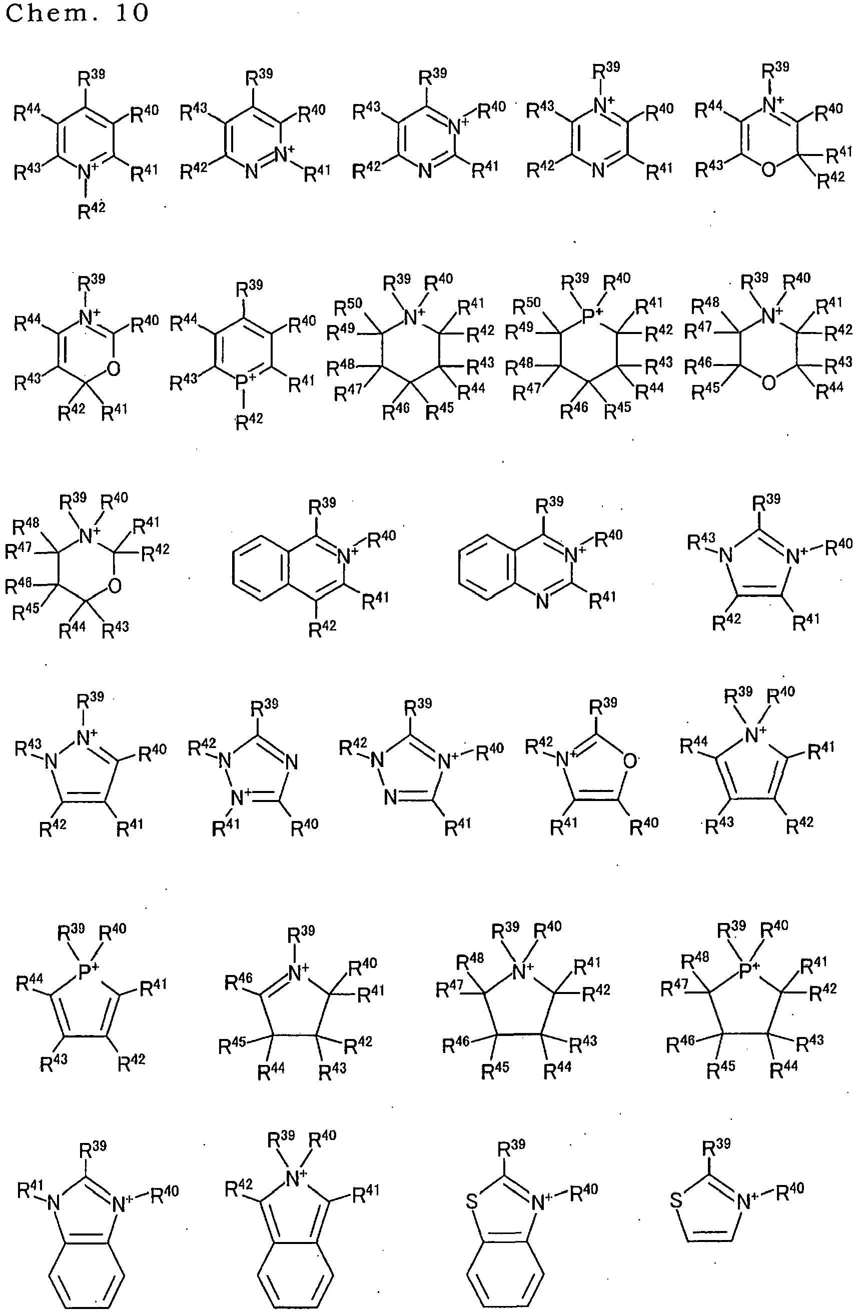 where R³⁹ to R⁵⁰ are a hydrogen atom(s), a saturated alkyl group(s), an unsaturated alkyl group(s), a cycloalkyl group(s), an aryl group(s), or an alkoxyalkyl group(s).

Among these, an imidazolium cation represented by General Formula (5), a pyridinium cation represented by General Formula (6), and a triazine derivative cation represented by General Formula (7) are preferable. 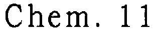 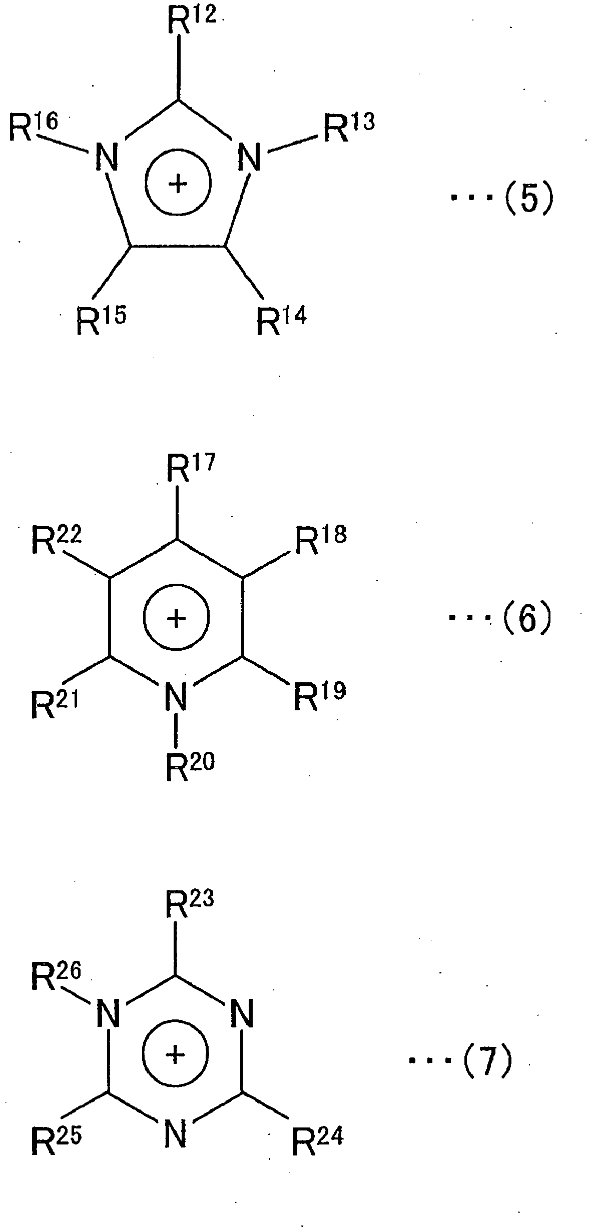 where R¹² to R²⁶ are a hydrogen atom(s), a saturated alkyl group(s), an unsaturated alkyl group(s), a cycloalkyl group(s), an aryl group(s), or an alkoxyalkyl group(s).

Among these, the imidazolium cation represented by General Formula (5) is more preferable. Ethylmethyl imidazolium cation and butylmethyl imidazolium cation are further preferable.

### (Anion Portion)

The anion portion of the ionic liquid producible by the production method of the present invention is preferably: an halide ion, triflate anion, tetrafluoroborate anion, hexafluorophosphate anion, nitrate ion, sulfate ion, N(CF₃SO₂)₂-, N(CN)₂-, C(CN)₃-, CH₃OSO₃-, (C₂H₅)₂PO₄-, N(C₂F₅SO₂)₂-, N(CF₃CO)₂-, N(CF₃SO₂)(CF₃CO)-, N(FSO₂)₂-, or CF₃COO-. More preferably, the anion portion may be N(CF₃CO)₂-, N(CN)₂-, , tetrafluoroborate anion, or hexafluorophosphate anion. Further preferably, the anion portion may be N(CN)₂-.

Embodiments of the present invention are explained in further details with reference to Examples dealt with in the following descriptions. It is obvious that the present invention is not limited to Examples described below , and can be embodied in various ways for details.

### Examples

### [Example 1]

### (Synthesis of Salt containing Cation Portion)

In 4-necked flask of 200ml, 16.42 g (0.200mol) of 1-methylimidazole, 20.37 g (0.220mol) of 1-chlorobutane, and 10.00g of toluene were added and stirred for 17 hours under reflux (approximately 106°C). After they were reacted in this way, a reaction solution was cooled (air-cooled) to 70°C or below. Then, 30.00g of ultra pure water was added therein, the resultant solution was water-cooled to room temperature.

After the cooling, an upper layer (toluene layer) of the solution was separated off from the solution by using a separating funnel. To a lower layer (water layer), 20.00g of toluene was further added. After the resultant solution was stirred for 30 min, an upper layer (toluene layer) was separated off from the solution by using a separating funnel, thereby remaining a water layer in which N-methyl-N'-butyl imidazolium chloride (hereinafter, referred to as [BMIm]Cl) being a cation portion of an ionic liquid was contained.

### (Anion-Exchange Reaction)

After the separation, 18.55g (0.200mol) of 96% sodium dicyanamide (NaDCA), and 70.00g of ultra pure water were added to the water layer thus remained. A resultant was stirred for 3 hours at room temperature in order to carry out an anion-exchange reaction. After the reaction, a resultant reaction solution was transferred to an egg-plant-shaped flask of 200ml and evaporated under reduced pressure at 60°C under 20mmHg for 2.5 hours, so as to remove water therefrom. In this way, a white slurry whose solvent was [BMIm]DCA was obtained.

### (Salt Removal Step)

After 20.40g of chloroform was added thereto, the white slurry thus obtained was cooled to a temperature in a range of approximately 0 to 5°C, and then stirred for 30 min, thereby crystallizing out sodium chloride (by-product) and unreacted sodium dicyanamide. The sodium chloride and unreacted sodium dicyanamide thus crystallized out was filtered out with a Buchner funnel having an internal diameter of 55mm. Crystals thus obtained were washed with chloroform (60.20g). A solution thus obtained was filtered to get a filtrate. The filtrate was then transferred to an egg-plant-shaped flask of 200ml and evaporated under reduced pressure at 50°C under 20mmHg for 2 hour, so as to remove chloroform therefrom. A concentrated solution thus obtained was mixed with 50.0g of chloroform. Then the solution was cooled down to a temperature approximately in a range of 0 to 5°C and stirred for 30 min so as to crystallize out crystals again. The crystals were then filtered out and washed with chloroform (34.50g). A solution thus obtained was filtered to get a filtrate. The filtrate was then transferred to an egg-plant-shaped flask of 200ml and evaporated under reduced pressure at 50°C under 20mmHg for 1 hour. The filtrate was then further evaporated under reduced pressure at 50°C under 1mmHg to 2mmHg for 1 hour.

### (Water Removing Step and Purifying Step)

To a concentrated liquid thus obtained, 4.81g (0.040mol) methyl orthoacetate (MOA) was added. A resultant mixture was stirred at 80 °C for 3 hours so as to react MOA with water not distilled off from an ionic liquid. The reaction caused hydrolysis of MOA with the water thereby giving methanol and methyl acetate. The methanol and methyl acetate thus obtained and unreacted MOA were evaporated off under reduced pressure at 50°C under 20mmHg for 30min. A concentrated liquid thus obtained was further evaporated under reduced pressure at 60°C under 1mmHg to 2mmHg for 2 hours. In this way, a target material, namely, N-methyl-N'-butyl imidazolium dicyanamide ([BMIm]DCA) was obtained with 90.4% yield (37.1g, 0.181mol). The resultant [BMIm]DCA was a light yellow liquid with chloride ion (Cl⁻) concentration of 2.66%.

### (Water Content Analysis of [BMIm]DCA)

Water content of [BMIm]DCA was measured by the Karl Fishcer test.

Water content before the addition of MOA was 0.95% (9500ppm), but water content after the addition of MOA was 0% (0ppm). Water content after methanol, methyl acetate and the unreacted MOA was evaporated off was 0.0061% (61ppm).

### [Example 2]

### (Synthesis of Salt Containing Cation Portion)

[BMIm]Cl was synthesized in the same manner as in Example 1.

### (Anion-Exchange Reaction)

A white slurry in which [BMIm]DCA served as a solvent was prepared in the same manner as in Example 1, except that evaporation under reduced pressure was carried out at 60°C under 20mmHg for 2 hours.

### (Salt Removing Step)

After 60.00g of acetone was added thereto, the white slurry was cooled to approximately 0°C to 5°C and stirred for 30 min, thereby crystallizing out sodium chloride and unreacted sodium dicyanamide. The sodium chloride and unreacted sodium dicyanamide thus crystallized out was filtered out with a Buchner funnel having an internal diameter of 55mm. Crystals thus obtained were washed with acetone (30.00g). A solution thus obtained was filtered to get a filtrate. The filtrate was then transferred to an egg-plant-shaped flask of 200ml and evaporated under reduced pressure at 50°C under 20mmHg for 1 hour. The filtrate was then further evaporated under reduced pressure at 50°C under 1mmHg to 2mmHg for 1 hour.

### (Water Removing Step and Purifying Step)

To a concentrated liquid thus obtained, 4.81g (0.040mol) methyl orthoacetate (MOA) was added. A resultant mixture was stirred at 80°C for 3 hours so as to react MOA with water not distilled off from an ionic liquid. The reaction caused hydrolysis of MOA with the water thereby giving methanol and methyl acetate. The methanol and methyl acetate thus obtained and unreacted MOA were evaporated off under reduced pressure at 50°C under 20mmHg for 30min. A concentrated liquid thus obtained was further evaporated under reduced pressure at 60°C under 1mmHg to 2mmHg for 2 hours. In this way, a target material, namely, N-methyl-N'-butyl imidazolium dicyanamide ([BMIm]DCA) was obtained with 97.2% yield (39.9g, 0.194mol). The resultant [BMIm]DCA was a light yellow liquid with chloride ion (Cl⁻) concentration of 0.16%

### (Water Content Analysis of [BMIm]DCA)

Water content of [BMIm]DCA was measured by the Karl Fishcer test.

Water content before the addition of MOA was 0.39% (3900ppm), but water content after the addition of MOA was 0% (0ppm). Water content after methanol, methyl acetate and the unreacted MOA was evaporated off was 0.0019% (19ppm).

### [Example 3]

### (Synthesis of Salt containing Cation Portion)

In 4-necked flask of 500ml, 151.9 g (1.850mol) of 1-methylimidazole, 188.4 g (2.035mol) of 1-chlorobutane, and 80.00g of toluene were added and stirred for 25 hours under reflux (approximately 106°C). After they were reacted in this way, a reaction solution was cooled (air-cooled) to 70°C or below. Then, 100.00g of ultra pure water was added therein, the resultant solution was water-cooled to room temperature.

After the cooling, an upper layer (toluene layer) of the solution was separated off from the solution by using a separating funnel. To a lower layer (water layer), 80.00g of toluene was further added. After the resultant solution was stirred for 30 min, an upper layer (toluene layer) was separated off from the solution by using a separating funnel, thereby remaining a water layer in which N-methyl-N'-butyl imidazolium chloride (hereinafter, referred to as [BMIm]Cl) being a cation portion of an ionic liquid was contained.

### (Anion-Exchange Reaction)

After the separation, the water layer (lower layer) was transferred to a 4-necked flask of 1000ml, to which 223.4g (2.035mol) of sodium tetrafluoroborate and 250.0g of ultra pure water were then added. A resultant mixture was stirred for 1 hour at room temperature in order to carry out an anion-exchange reaction. After the reaction, a lower layer (water layer) was separated out by using a separating funnel. An upper layer was mixed with 50.0g of ultra pure water and stirred for 30min. The stirring emulsified a resultant reaction liquid. The emulsified reaction liquid was then filtered. With 50.0g of acetone, a resultant filtrate was transferred to an egg-plant-shaped flask of 500ml and evaporated under reduced pressure at 60°C under 20mmHg for 3.5 hours, so as to distill off water and acetone. In this way, a light yellow slurry whose solvent was N-methyl-N'-butyl imidazolium Tetrafluoroborate ([BMIm]BF4) was obtained.

### (Salt Removal Step)

After 100.0g of acetone was added thereto, the light yellow slurry thus obtained was cooled to a temperature in a range of approximately 0 to 5°C, and then stirred for 30 min, thereby crystallizing out sodium chloride (by-product) and unreacted sodium tetrafluoroborate. The sodium chloride and unreacted sodium dicyanamide thus crystallized out was filtered out with a Buchner funnel having an internal diameter of 55mm. Crystals thus obtained were washed with acetone (70.0g). A solution thus obtained was filtered to get a filtrate. The filtrate was then transferred to an egg-plant-shaped flask of 500ml and evaporated under reduced pressure at 60°C under 20mmHg for 3 hour. In this way, 379.8g of a concentrated solution containing the target [BMIm]BF4 was obtained. The concentrated solution had water content of 0.1284% (1284ppm).

### (Water Removing Step and Purifying Step)

30.0g of the concentrated solution was transferred to an egg-plant-shaped flask of 50ml and mixed with 0.45g (0.00428mol) of methyl orthoformate (OFM). A resultant mixture was stirred at 80°C for 4 hours so as to react OFM with water not distilled off from an ionic liquid. The reaction caused hydrolysis of OFM with the water thereby giving methanol and methyl formate. The methanol and methyl formate thus obtained and unreacted OFM were evaporated off under reduced pressure at 60°C under 20mmHg for 1 hour. A concentrated liquid thus obtained was further evaporated under reduced pressure at 60°C under 1mmHg to 2mmHg for 2 hours. In this way, a target material, [BMIm]BF4 was obtained as a light yellow liquid.

### (Water Content Analysis of [BMIm]BF4)

Water content of [BMIm]BF4 was measured by the Karl Fishcer test. Water content after the addition of OFM was 0.0112% (112ppm). Water content after methanol, methyl formate and the unreacted OFM was evaporated off was 0.0066% (66ppm).

### [Example 4]

### (Water Removing Step and Purifying Step)

30.0g of the concentrated solution containing [BMIm]BF4 obtained in Example 3 was transferred to an egg-plant-shaped flask of 50ml and mixed with 0.81g (0.00428mol) of n-Propyl orthoformate (OFNP). A resultant mixture was stirred at 80°C for 3 hours so as to react OFNP with water not distilled off from an ionic liquid. The reaction caused hydrolysis of OFNP with the water thereby giving methanol and n-propyl formate. The methanol and n-propyl formate thus obtained and unreacted OFNP were evaporated off under reduced pressure at 60°C under 20mmHg for 1 hour. A concentrated liquid thus obtained was further evaporated under reduced pressure at 60°C under 1mmHg to 2mmHg for 3 hours. In this way, a target material, [BMIm]BF4 was obtained as a light yellow liquid.

### (Water Content Analysis of [BMIm]BF4)

Water content of [BMIm]BF4 was measured by the Karl Fishcer test. Water content after the addition of OFNP was 0.0037% (37ppm). Water content after methanol, n-propyl formate and the unreacted OFNP was evaporated off was 0.0046% (46ppm).

### [Example 5]

### (Water Removing Step and Purifying Step)

30.0g of the concentrated solution containing [BMIm]BF4 obtained in Example 3 was transferred to an egg-plant-shaped flask of 50ml and mixed with 0.69g (0.00425mol) of ethyl orthoacetate (EOA). A resultant mixture was stirred at 80°C for 3 hours so as to react EOA with water not distilled off from an ionic liquid. The reaction caused hydrolysis of EOA with the water thereby giving ethanol and ethyl acetate. The ethanol and ethyl acetate thus obtained and unreacted EOA were evaporated off under reduced pressure at 60°C under 20mmHg for 1 hour. A concentrated liquid thus obtained was further evaporated under reduced pressure at 60°C under 1mmHg to 2mmHg for 3 hours. In this way, a target material, [BMIm]BF4 was obtained as a light yellow liquid.

### (Water Content Analysis of [BMIm]BF4)

Water content of [BMIm]BF4 was measured by the Karl Fishcer test. Water content after the addition of EOA was 0.0042% (42ppm). Water content after ethanol, ethyl acetate and the unreacted EOA was evaporated off was 0.0031% (31ppm).

### [Example 6]

### (Water Removing Step and Purifying Step)

30.0g of the concentrated solution containing [BMIm]BF4 obtained in Example 3 was transferred to an egg-plant-shaped flask of 50ml and mixed with 0.63g (0.00425mol) of methyl ortho n-butyrate (MOB). A resultant mixture was stirred at 80°C for 3 hours so as to react MOB with water not distilled off from an ionic liquid. The reaction caused hydrolysis of MOB with the water thereby giving methanol and methyl n-butyrate. The methanol and methyl n-butyrate thus obtained and unreacted MOB were evaporated off under reduced pressure at 60°C under 20mmHg for 1 hour. A concentrated liquid thus obtained was further evaporated under reduced pressure at 60°C under 1mmHg to 2mmHg for 2 hours. In this way, a target material, [BMIm]BF4 was obtained as a light yellow liquid.

### (Water Content Analysis of [BMIm]BF4)

Water content of [BMIm]BF4 was measured by the Karl Fishcer test. Water content after the addition of MOB was 0.0038% (38ppm). Water content after methanol, methyl n-butyrate and the unreacted MOB was evaporated off was 0.0072% (72ppm).

### [Example 7]

### (Water Removing Step and Purifying Step)

30.0g of the concentrated solution containing [BMIm]BF4 obtained in Example 3 was transferred to an egg-plant-shaped flask of 50ml and mixed with 0.63g (0.00425mol) of ethyl orthoformate (OFE). A resultant mixture was stirred at 80°C for 3 hours so as to react OFE with water not distilled off from an ionic liquid. The reaction caused hydrolysis of OFE with the water thereby giving ethanol and ethyl formate. The ethanol and ethyl formate thus obtained and unreacted OFE were evaporated off under reduced pressure at 60°C under 20mmHg for 1 hour. A concentrated liquid thus obtained was further evaporated under reduced pressure at 60°C under 1mmHg to 2mmHg for 3 hours. In this way, a target material, [BMIm]BF4 was obtained as a light yellow liquid.

### (Water Content Analysis of [BMIm]BF4)

Water content of [BMIm]BF4 was measured by the Karl Fishcer test. Water content after the addition of OFE was 0.0085% (85ppm). Water content after ethanol, ethyl formate and the unreacted OFE was evaporated off was 0.0054% (54ppm).

### [Example 8]

### (Water Removing Step and Purifying Step)

30.0g of the concentrated solution containing [BMIm]BF4 obtained in Example 3 was transferred to an egg-plant-shaped flask of 50ml and mixed with 0.87g (0.00426mol) of methyl ortho n-octanoate (OEC8). A resultant mixture was stirred at 80°C for 3 hours so as to react OEC8 with water not distilled off from an ionic liquid. The reaction caused hydrolysis of OEC8 with the water thereby giving methanol and methyl n-octanoate. The methanol and methyl n-octanoate thus obtained and unreacted OEC8 were evaporated off under reduced pressure at 60°C under 20mmHg for 1 hour. A concentrated liquid thus obtained was further evaporated under reduced pressure at 60°C under 1mmHg to 2mmHg for 3 hours. In this way, a target material, [BMIm]BF4 was obtained as a light yellow liquid.

### (Water Content Analysis of [BMIm]BF4)

Water content of [BMIm]BF4 was measured by the Karl Fishcer test. Water content after the addition of OEC8 was 0.0041% (41ppm). Water content after methanol, methyl n-octanoate, and the unreacted OEC8 was evaporated off was 0.0153% (153ppm).

### [Example 9]

### (Water Removing Step and Purifying Step)

30.0g of the concentrated solution containing [BMIm]BF4 obtained in Example 3 was transferred to an egg-plant-shaped flask of 50ml and mixed with 0.45g (0.00432mol) of 2,2-Dimethoxypropane (DMP). A resultant mixture was stirred at 80°C for 6 hours so as to react DMP with water not distilled off from an ionic liquid. The reaction caused hydrolysis of DMP with the water thereby giving methanol and acetone. The methanol and acetone thus obtained and unreacted DMP were evaporated off under reduced pressure at 60°C under 20mmHg for 1 hour. A concentrated liquid thus obtained was further evaporated under reduced pressure at 60°C under 1mmHg to 2mmHg for 3 hours. In this way, a target material, [BMIm]BF4 was obtained as a light yellow liquid.

### (Water Content Analysis of [BMIm]BF4)

Water content of [BMIm]BF4 was measured by the Karl Fishcer test. Water content after the addition of DMP was 0.0189% (189ppm). Water content after methanol, acetone, and the unreacted DMP was evaporated off was 0.0164% (164ppm).

### [Example 10]

### (Water Removing Step and Purifying Step)

30.0g of the concentrated solution containing [BMIm]BF4 obtained in Example 3 was transferred to an egg-plant-shaped flask of 50ml and mixed with 0.51g (0.00432mol) of 1,1-Diethoxyethane (DEE). A resultant mixture was stirred at 80°C for 6 hours so as to react DEE with water not distilled off from an ionic liquid. The reaction caused hydrolysis of DEE with the water thereby giving ethanol and acetaldehyde. The ethanol and acetaldehyde thus obtained and unreacted DEE were evaporated off under reduced pressure at 60°C under 20mmHg for 1 hour. A concentrated liquid thus obtained was further evaporated under reduced pressure at 60°C under 1mmHg to 2mmHg for 3 hours. In this way, a target material, [BMIm]BF4 was obtained as a light yellow liquid.

### (Water Content Analysis of [BMIm]BF4)

Water content of [BMIm]BF4 was measured by the Karl Fishcer test. Water content after ethanol, acetaldehyde, and the unreacted DEE was evaporated off was 0.0100% (100ppm)

### [Example 11]

### (Water Removing Step and Purifying Step)

30.0g of the concentrated solution containing [BMIm]BF4 obtained in Example 3 was transferred to an egg-plant-shaped flask of 50ml and mixed with 0.39g (0.00433mol) of 1,1-Dimethoxyethane (DME). A resultant mixture was stirred at 80°C for 3 hours so as to react DME with water not distilled off from an ionic liquid. The reaction caused hydrolysis of DME with the water thereby giving methanol and acetaldehyde. The methanol and acetaldehyde thus obtained and unreacted DME were evaporated off under reduced pressure at 60°C under 20mmHg for 1 hour. A concentrated liquid thus obtained was further evaporated under reduced pressure at 60°C under 1 mmHg to 2mmHg for 3 hours. In this way, a target material, [BMIm]BF4 was obtained as a light yellow liquid.

### (Water Content Analysis of [BMIm]BF4)

Water content of [BMIm]BF4 was measured by the Karl Fishcer test. Water content after methanol, acetaldehyde, and the unreacted DME was evaporated off was 0.0058% (58ppm).

### [Example 12]

### (Synthesis of Salt containing Cation Portion)

In 4-necked flask of 500ml, 82.1 g (1.000mol) of 1-methylimidazole, 101.8 g (1.100mol) of 1-chlorobutane, and 50.0g of toluene were added and stirred for 15 hours under reflux (approximately 106°C). After they were reacted in this way, a reaction solution was cooled (air-cooled) to 70°C or below. Then, 100.0g of ultra pure water was added therein, the resultant solution was water-cooled to room temperature.

After the cooling, an upper layer (toluene layer) of the solution was separated off from the solution by using a separating funnel. To a lower layer (water layer), 100.0g of toluene was further added. After the resultant solution was stirred for 30 min, an upper layer (toluene layer) was separated off from the solution by using a separating funnel, thereby remaining a water layer in which [BMIm]Cl being a cation portion of an ionic liquid was contained.

### (Anion-Exchange Reaction)

After the separation, 53.8g of the water layer containing [BMIm]Cl (corresponding to 0.200mol of [BMIm]Cl) was transferred to a 300ml conical flask, and mixed with 63.2g (0.220mol) of lithium bis(trifluoromethanesulfonyl)imide and 50.0g of ultra pure water. A resultant mixture was stirred for 30min at room temperature in order to carry out an anion-exchange reaction. After the reaction, an upper layer (water layer) was separated out by using a separating funnel. A lower layer was mixed with 50.0g of ultra pure water and stirred for 30min at room temperature. Then, an upper layer (water layer) was separated out by using a separating funnel. A lower layer containing the cation portion of the ionic liquid was transferred to an egg-plant-shaped flask of 100ml and evaporated under reduced pressure at 60°C under 20mmHg for 1 hour, so as to distill off water. In this way, a light yellow solution whose solvent was N-methyl-N'-butyl imidazolium bis(trifluoromethanesulfonyl)imide ([BMIm]NTf2) was obtained.

### (Salt Removal Step)

After 50.0g of acetone was added thereto, the light yellow solution thus obtained was cooled to a temperature in a range of approximately 0 to 5°C, and then stirred for 30 min, thereby trying to crystallize out lithium chloride (by-product) and unreacted lithium bis(trifluoromethanesulfonyl)imide. Even though the crystallization yielded no crystals, the resultant solution was filtered with a Buchner funnel having an internal diameter of 55mm, and then washed with 20.0g of acetone. A filtrate obtained from the filtration was then transferred to an egg-plant-shaped flask of 200ml and evaporated under reduced pressure at 60°C under 20mmHg for 2 hours, and then further evaporated under reduced pressure at 60°C under 1mmHg to 2mmHg for 1 hour. In this way, 73.8g of a concentrated solution containing the target [BMIm]NTf2 was obtained. The concentrated solution had water content of 0.0111% (111ppm).

### (Water Removing Step and Purifying Step)

Then, 73.8g of the concentrated solution thus obtained was mixed with 0.20g (0.00166mol) of methyl orthoacetate (MOA). A resultant mixture was stirred at 80°C for 3 hours so as to react MOA with water not distilled off from an ionic liquid. The reaction caused hydrolysis of MOA with the water thereby giving methanol and methyl acetate. The methanol and methyl acetate thus obtained and unreacted MOA were evaporated off under reduced pressure at 60°C under 20mmHg for 1 hour. A concentrated liquid thus obtained was further evaporated under reduced pressure at 60°C under 1mmHg to 2mmHg for 2 hours. In this way, a target material, [BMIm]NTf2 was obtained as a light yellow liquid.

### (Water Content Analysis of [BMIm]NTf2)

Water content of [BMIm]NTf2 was measured by the Karl Fishcer test. Water content after the addition of MOA was 0.0032% (32ppm). Water content after methanol, methyl acetate and the unreacted MOA was evaporated off was 0.0037% (37ppm).

### [Example 13]

### (Anion-Exchange Reaction)

Then, 79.9g of water layer containing [BMIm]Cl (corresponding to 0.296mol of [BMIm]Cl) obtained in Example 12 was transferred to a 300ml conical flask, and mixed with 36.2g (0.330mol) of sodium tetrafluoroborate and 50.0g of ultra pure water. A resultant mixture was stirred for 30min at room temperature in order to carry out an anion-exchange reaction. After the reaction, 50.0g of ultra pure water was further added thereto. Then, an upper layer (water layer) was separated out by using a separating funnel. A lower layer was mixed with 20.0g of ultra pure water and stirred for 30min at room temperature. After the stirring, the liquid was in a single-layer state without dividing into multi layers. Together with 20.0g of accetone, the liquid, which contained the cation portion of the ionic liquid, was transferred to an egg-plant-shaped flask of 100ml and evaporated under reduced pressure at 60°C under 20mmHg for 1 hour, so as to distill off water. In this way, a light yellow slurry whose solvent was N-methyl-N'-butyl imidazolium tetrafluoroborate ([BMIm]BF4) was obtained.

### (Salt Removal Step)

After 50.0g of acetone was added thereto, the light yellow slurry thus obtained was cooled to a temperature in a range of approximately 0 to 5°C, and then stirred for 30 min, thereby trying to crystallize out sodium chloride (by-product) and unreacted sodium tetrafluoroborate. The resultant solution was filtered with a Buchner funnel having an internal diameter of 55mm, and then washed with 20.0g of acetone. A filtrate obtained from the filtration was then transferred to an egg-plant-shaped flask of 100ml and evaporated under reduced pressure at 60°C under 20mmHg for 2 hours, and then further evaporated under reduced pressure at 60°C under 1mmHg to 2mmHg for 1 hour. In this way, 32.7g of a concentrated solution containing the target [BMIm]BF4 was obtained. The concentrated solution had water content of 0.0233% (233ppm).

### (Water Removing Step and Purifying Step)

Then, 32.7g of the concentrated solution thus obtained was mixed with 0.20g (0.00166mol) of methyl orthoacetate (MOA). A resultant mixture was stirred at 80°C for 3 hours so as to react MOA with water not distilled off from an ionic liquid. The reaction caused hydrolysis of MOA with the water thereby giving methanol and methyl acetate. The methanol and methyl acetate thus obtained and unreacted MOA were evaporated off under reduced pressure at 60°C under 20mmHg for 1 hour. A concentrated liquid thus obtained was further evaporated under reduced pressure at 60°C under 1mmHg to 2mmHg for 2 hours. In this way, a target material, [BMIm]BF4 was obtained as a light yellow liquid.

### (Water Content Analysis of [BMIm]BF4)

Water content of [BMIm]BF4 was measured by the Karl Fishcer test. Water content after the addition of MOA was 0.0082% (82ppm). Water content after methanol, methyl acetate and the unreacted MOA was evaporated off was 0.0087% (87ppm).

### [Example 14]

### (Anion-Exchange Reaction)

Then, 134.5g of water layer containing [BMIm]Cl (corresponding to 0.500mol of [BMIm]Cl) obtained in Example 12 was transferred to a 300ml conical flask, and mixed with 49.0g (0.528mol) of 96% sodium dicyanamide (NaDCA) and 150.0g of ultra pure water. A resultant mixture was stirred for 30min at room temperature in order to carry out an anion-exchange reaction. After the reaction, the liquid was in a single-layer state without dividing into multi layers. the liquid, which contained the cation portion of the ionic liquid, was transferred to an egg-plant-shaped flask of 500ml and evaporated under reduced pressure at 60°C under 20mmHg for 1 hour, so as to distill off water. In this way, a light yellow slurry whose solvent was N-methyl-N'-butyl imidazolium dicyanamide ([BMIm]DCA) was obtained.

### (Salt Removal Step)

After 100.0g of acetone was added thereto, the light yellow slurry thus obtained was cooled to a temperature in a range of approximately 0 to 5°C, and then stirred for 30 min, thereby crystallizing out sodium chloride (by-product) and unreacted sodium dicyanamide. The crystals were filtered out with a Buchner funnel having an internal diameter of 55mm, and then washed with 100.0g of acetone. A filtrate obtained from the filtration was then transferred to an egg-plant-shaped flask of 300ml and evaporated under reduced pressure at 60°C under 20mmHg for 2 hours, and then further evaporated under reduced pressure at 60°C under 1mmHg to 2mmHg for 1 hour. In this way, 106.2g of a concentrated solution containing the target [BMIm]DCA was obtained. The concentrated solution had water content of 0.2794% (2794ppm).

### (Water Removing Step and Purifying Step)

Then, 106.2g of the concentrated solution thus obtained was mixed with 4.00g (0.0333mol) of methyl orthoacetate (MOA). A resultant mixture was stirred at 80°C for 3 hours so as to react MOA with water not distilled off from an ionic liquid. The reaction caused hydrolysis of MOA with the water thereby giving methanol and methyl acetate. The methanol and methyl acetate thus obtained and unreacted MOA were evaporated off under reduced pressure at 60°C under 20mmHg for 1 hour. A concentrated liquid thus obtained was further evaporated under reduced pressure at 60°C under 1mmHg to 2mmHg for 2 hours. In this way, a target material, [BMIm]DCA was obtained as a light yellow liquid.

### (Water Content Analysis of [BMIm]NTf2)

Water content of [BMIm]NTf2 was measured by the Karl Fishcer test. Water content after the addition of MOA was 0.0129% (129ppm). Water content after methanol, methyl acetate and the unreacted MOA was evaporated off was 0.0089% (89ppm).

### [Example 15]

### (Synthesis of Salt containing Cation Portion)

In 4-necked flask of 300ml, 39.3 g (0.150mol) of triphenylphosphine, 31.9 g (0.165mol) of 1-bromooctane, and 100.0g of toluene were added and stirred for 14 hours under reflux (approximately 106°C). After they were reacted in this way, a reaction solution was cooled (air-cooled) to 70°C or below.

After the cooling, an upper layer (toluene layer) of the solution was separated off from the solution by using a separating funnel. To a lower layer (organic layer), 50.0g of toluene was further added. After the resultant solution was stirred for 30 min, an upper layer (toluene layer) was separated off from the solution by using a separating funnel, thereby remaining an organic layer in which triphenyl n-octylphosphonium bromide ([TPPO]Br) being a cation portion of an ionic liquid was contained.

### (Anion-Exchange Reaction)

After the separation, 82.2g of the solution containing [TPPO]Br (corresponding to 0.150mol of [TPPO]Br) was transferred to a 300ml egg-plant-shaped flask, and mixed with 47.4g (0.165mol) of lithium bis (trifluoromethanesulfonyl) imide and 50.0g of ultra pure water. A resultant mixture was stirred for 1 hour at room temperature in order to carry out an anion-exchange reaction. After the reaction, a lower layer (water layer) was separated out by using a separating funnel. Together with 20.0g of acetone, an upper layer containing the cation portion of the ionic liquid was transferred to an egg-plant-shaped flask of 200ml and evaporated under reduced pressure at 60°C under 20mmHg for 1 hour, so as to distill off a solvent. In this way, a light yellow solution containing triphenyl n-octylphosphonium bis (trifluoromethanesulfonyl) imide ([TPPO]NTf2) was obtained.

### (Salt Removal Step)

After 50.0g of acetone was added thereto, the light yellow solution thus obtained was cooled to a temperature in a range of approximately 0 to 5°C, and then stirred for 30 min, thereby trying to crystallize out lithium bromide (by-product) and unreacted lithium bis (trifluoromethanesulfonyl) imide. Even though the crystallization yielded no crystals, the resultant solution was filtered with a Buchner funnel having an internal diameter of 55mm, and then washed with 20.0g of acetone. A filtrate obtained from the filtration was then transferred to an egg-plant-shaped flask of 200ml and evaporated under reduced pressure at 60°C under 20mmHg for 2 hours, and then further evaporated under reduced pressure at 60°C under 1mmHg to 2mmHg for 1 hour. In this way, 78.2g of a concentrated solution containing the target [TPPO]NTf2 was obtained. The concentrated solution had water content of 0.0732% (732ppm).

### (Water Removing Step and Purifying Step)

Then, 78.2g of the concentrated solution thus obtained was mixed with 4.00g (0.0333mol) of methyl orthoacetate (MOA). A resultant mixture was stirred at 80°C for 3 hours so as to react MOA with water not distilled off from an ionic liquid. The reaction caused hydrolysis of MOA with the water thereby giving methanol and methyl acetate. The methanol and methyl acetate thus obtained and unreacted MOA were evaporated off under reduced pressure at 60°C under 20mmHg for 1 hour. A concentrated liquid thus obtained was further evaporated under reduced pressure at 60°C under 1mmHg to 2mmHg for 2 hours. In this way, a target material, [TPPO]NTf2 was obtained as a light yellow liquid.

### (Water Content Analysis of [TPPO]NTf2)

Water content of [TPPO]NTf2 was measured by the Karl Fishcer test. Water content after the addition of MOA was 0.0369% (369ppm). Water content after methanol, methyl acetate and the unreacted MOA was evaporated off was 0.0027% (27ppm).

### [Example 16]

### (Synthesis of Salt containing Cation Portion)

In 4-necked flask of 300ml, 15.8 g (0.200mol) of pyridine, 26.5 g (0.220mol) of 1-chlorohexane, and 50.0g of toluene were added and stirred for 22 hours under reflux (approximately 106°C). After they were reacted in this way, a reaction solution was cooled (air-cooled) to 70°C or below. Then, 50.5g of ultra pure water was added therein, the resultant solution was water-cooled to room temperature.

After the cooling, an upper layer (toluene layer) of the solution was separated off from the solution by using a separating funnel. To a lower layer (organic layer), 50.0g of toluene was further added. After the resultant solution was stirred for 30 min, an upper layer (toluene layer) was separated off from the solution by using a separating funnel, thereby remaining a water layer in which N-n-hexyl pyridinium chloride (hereinafter, referred to as [HPy]Cl) being a cation portion of an ionic liquid was contained.

### (Anion-Exchange Reaction)

After the separation, 126.8g of the solution containing [HPy]Cl (corresponding to 0.200mol of [HPy]Cl) was transferred to a 300ml egg-plant-shaped flask, and mixed with 63.2g (0.220mol) of lithium bis (trifluoromethanesulfonyl) imide. A resultant mixture was stirred for 1 hour at room temperature in order to carry out an anion-exchange reaction. After the reaction, an upper layer (water layer) was separated out by using a separating funnel. A lower layer was mixed with 50.0g of ultra pure water and stirred for 30min at room temperature. Then, an upper layer (water layer) was separated out by using a separating funnel. A lower layer containing the cation portion of the ionic liquid was transferred to an egg-plant-shaped flask of 200ml and evaporated under reduced pressure at 60°C under 20mmHg for 1 hour, so as to distill off a solvent. In this way, a light yellow solution containing N-n-hexyl pyridinium bis (trifluoromethanesulfonyl) imide ([HPy]NTf2) was obtained.

### (Salt Removal Step)

After 50.0g of acetone was added thereto, the light yellow solution thus obtained was cooled to a temperature in a range of approximately 0 to 5°C, and then stirred for 30 min, thereby trying to crystallize out lithium chloride (by-product) and unreacted lithium bis (trifluoromethanesulfonyl) imide. Even though the crystallization yielded no crystals, the resultant solution was filtered with a Buchner funnel having an internal diameter of 55mm, and then washed with 20.0g of acetone. A filtrate obtained from the filtration was then transferred to an egg-plant-shaped flask of 200ml and evaporated under reduced pressure at 60°C under 20mmHg for 3 hours, and then further evaporated under reduced pressure at 60°C under 1mmHg to 2mmHg for 1 hour. In this way, 93.4g of a concentrated solution containing the target [HPy]NTf2 was obtained. The concentrated solution had water content of 0.0069% (69ppm).

### (Water Removing Step and Purifying Step)

Then, 93.4g of the concentrated solution thus obtained was mixed with 0.20g (0.00166mol) of methyl orthoacetate (MOA). A resultant mixture was stirred at 80°C for 3 hours so as to react MOA with water not distilled off from an ionic liquid. The reaction caused hydrolysis of MOA with the water thereby giving methanol and methyl acetate. The methanol and methyl acetate thus obtained and unreacted MOA were evaporated off under reduced pressure at 60°C under 20mmHg for 1 hour. A concentrated liquid thus obtained was further evaporated under reduced pressure at 60°C under 1mmHg to 2mmHg for 2 hours. In this way, a target material, [HPy]NTf2 was obtained as a light yellow liquid.

### (Water Content Analysis of [HPy]NTf2)

Water content of [HPy]NTf2 was measured by the Karl Fishcer test. Water content after the addition of MOA was 0.0000% (0ppm). Water content after methanol, methyl acetate and the unreacted MOA was evaporated off was 0.0000% (0ppm).

### [Example 17]

### (Synthesis of Salt containing Cation Portion)

In 4-necked flask of 200ml, 20.2 g (0.200mol) of triethylamine, 36.3 g (0.220mol) of 1-bromohexane, and 50.0g of toluene were added and stirred for 23 hours under reflux (approximately 106°C). After they were reacted in this way, a reaction solution was cooled (air-cooled) to 70°C or below. Then, 50.0g of ultra pure water was added therein, the resultant solution was water-cooled to room temperature.

After the cooling, an upper layer (toluene layer) of the solution was separated off from the solution by using a separating funnel. To a lower layer (water layer), 50.0g of toluene was further added. After the resultant solution was stirred for 30 min, an upper layer (toluene layer) was separated off from the solution by using a separating funnel, thereby remaining an organic layer in which triethyl n-hexyl ammonium bromide (hereinafter, referred to as [TEHA]Br) being a cation portion of an ionic liquid was contained.

### (Anion-Exchange Reaction)

After the separation, 94.0g of the solution containing [TEHA]Br (corresponding to 0.200mol of [TEHA]Br) was transferred to a 300ml conical flask, and mixed with 63.2g (0.220mol) of lithium bis(trifluoromethanesulfonyl)imide. A resultant mixture was stirred for 1 hour at room temperature in order to carry out an anion-exchange reaction. After the reaction, an upper layer (water layer) was separated out by using a separating funnel. A lower layer was mixed with 50.0g of ultra pure water and stirred for 30min at room temperature. Then, an upper layer (water layer) was separated out by using a separating funnel. A lower layer containing the cation portion of the ionic liquid was transferred to an egg-plant-shaped flask of 200ml and evaporated under reduced pressure at 60°C under 20mmHg for 1 hour, so as to distill off a solvent. In this way, a light yellow solution containing triethyl n-hexyl ammonium bis (trifluoromethanesulfonyl) imide ([TEHA]NTf2) was obtained.

### (Salt Removal Step)

After 50.0g of acetone was added thereto, the light yellow solution thus obtained was cooled to a temperature in a range of approximately 0 to 5°C, and then stirred for 30 min, thereby trying to crystallize out lithium **bromide** (by-product) and unreacted lithium bis (trifluoromethanesulfonyl) imide. Even though the crystallization yielded no crystals, the resultant solution was filtered with a Buchner funnel having an internal diameter of 55mm, and then washed with 20.0g of acetone. A filtrate obtained from the filtration was then transferred to an egg-plant-shaped flask of 200ml and evaporated under reduced pressure at 60°C under 20mmHg for 2 hours, and then further evaporated under reduced pressure at 60°C under 1mmHg to 2mmHg for 1 hour. In this way, 73.7g of a concentrated solution containing the target [TEHA]NTf2 was obtained. The concentrated solution had water content of 0.0085% (85ppm).

### (Water Removing Step and Purifying Step)

Then, 73.7g of the concentrated solution thus obtained was mixed with 0.20g (0.00166mol) of methyl orthoacetate (MOA). A resultant mixture was stirred at 80°C for 3 hours so as to react MOA with water not distilled off from an ionic liquid. The reaction caused hydrolysis of MOA with the water thereby giving methanol and methyl acetate. The methanol and methyl acetate thus obtained and unreacted MOA were evaporated off under reduced pressure at 60°C under 20mmHg for 1 hour. A concentrated liquid thus obtained was further evaporated under reduced pressure at 60°C under 1mmHg to 2mmHg for 2 hours. In this way, a target material, [TEHA]NTf2 was obtained as a light yellow liquid.

### (Water Content Analysis of [TEHA]NTf2)

Water content of [TEHA]NTf2 was measured by the Karl Fishcer test. Water content after the addition of MOA was 0.0030% (30ppm). Water content after methanol, methyl acetate and the unreacted MOA was evaporated off was 0.0042% (42ppm).

### [Comparative Example 1]

Here, 30.0g of the concentrated liquid, obtained in Example 3, containing [BMIm]BF4 and having a water content of 0.1284% (1284ppm) was transferred to a 50ml egg-plant-shaped flask, and evaporated under reduced pressure at 60°C under 1mmHg to 2mmHg for 6 hours. In this way, target [BMIm]BF4 was obtained as a light yellow liquid.

Water content of [BMIm]BF4 was measured by the Karl Fishcer test. Water content thereof after the evaporation under reduced pressured was 0.0389% (389ppm).

### [Comparative Example 2]

Here, [BMIm]DCA obtained in Example 14 was mixed with ultra pure water to a water content of 0.3919% (3919ppm). 30.0g of the diluted [BMIm]DCA was evaporated under reduced pressure at 60°C under 1mmHg to 2mmHg for 8 hours. In this way, target [BMIm]DCA was obtained as a light yellow liquid.

Water content of [BMIm]DCA was measured by the Karl Fishcer test. Water content thereof after the evaporation under reduced pressured was 0.1197% (1197 ppm)

### [Comparative Example 3]

Here, [BMIm]DCA obtained in Example 14 was mixed with ultra pure water to a water content of 0.3528% (3528ppm). 20.0g of the diluted [BMIm]DCA was mixed with 1.0g of a molecular sieve (3A). After 10-min stirring, the diluted [BMIm]DCA was let stand over night. Then, the diluted [BMIm]DCA was stirred for one night. In this way, dehydration was carried out.

Water content of [BMIm]DCA was measured by the Karl Fishcer test. Water content thereof after letting stand over night was 0.2583% (2583ppm). Water content thereof after the one-night stirring was 0.1351% (1351ppm).

### [Comparative Example 4]

Here, [BMIm]DCA obtained in Example 14 was mixed with ultra pure water to a water content of 0.3528% (3528ppm). 20.0g of the diluted [BMIm]DCA was mixed with 1.0g of silica gel (blue, medium particle size). After 10-min stirring, the diluted [BMIm]DCA was let stand over night. Then, the diluted [BMIm]DCA was stirred for one night. In this way, dehydration was carried out.

Water content of [BMIm]DCA was measured by the Karl Fishcer test. Water content thereof after letting stand over night was 0.3707% (3707ppm). Water content thereof after the one-night stirring was 0.3803% (3803ppm).

As described above, a production method according to the present invention includes removing water from a molten salt solution, the step of removing the water including adding a compound in the molten salt solution, the compound being represented by General Formula (1).

With this, the water removal can be performed in much shorter time, and can be performed without the use of an adsorbent agent such as a molecular sieve, silica, or the like.

That is, the production method according to the present invention is applicable to production of any kind of ionic liquids, and makes it possible to remove water from the molten salt solution economically, easily, and quickly.

## Claims

1. A method for producing an ionic liquid from a molten salt solution containing a molten salt constituted by combining the cation portion and the anion portion, the ionic liquid containing the molten salt as a main component, the method comprising:
removing water from the molten salt solution, the step of removing the water including adding a compound in the molten salt solution, the compound being represented by General Formula (1): 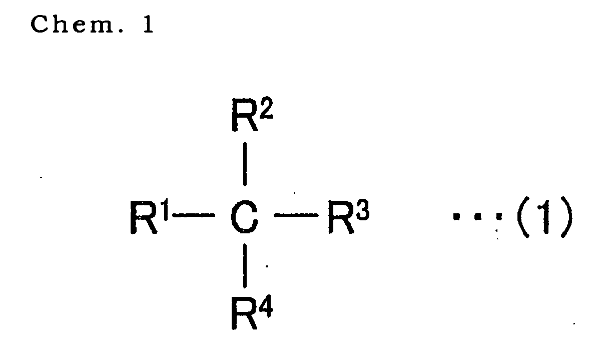 where at least two of R¹ to R⁴ are a C1 to C8 alkoxy group(s), the rest of R¹ to R⁴ are a hydrogen atom(s) or a C1 to C8 alkyl group(s), and
purifying a mixture obtained by the step of removing the water, the step of purifying removing at least one of a compound represented by General Formula (2) and an alcohol represented by General Formula (3),
General Formula (2) being as follows: 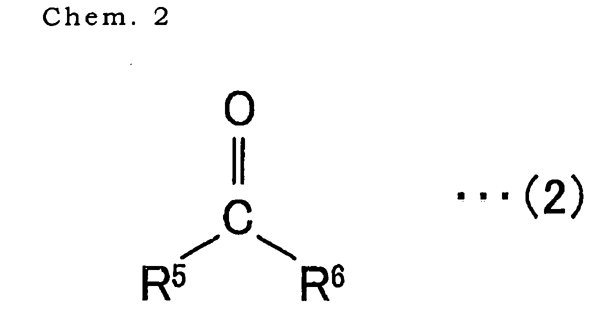 where R⁵ and R⁶ are a hydrogen atom(s), a C1 to C8 alkyl group(s), or a C1 to C8 alkoxy group(s), and
General Formula (3) being as follows:
Chem. 3
**R⁷⁻OH** (3)
where R⁷ is a C1 to C8 alkyl group.

2. The method according to claim 1, further comprising:
synthesizing the molten salt,
wherein:
in the step of removing the water, the compound is added to the molten salt solution obtained in the step of synthesizing the molten salt.

3. The method as set forth in claim lor 2, wherein:
the compound represented by General Formula (1) is an acetal or an orthoester compound.

4. The method as set forth in claim 3, wherein:
the acetal or the orthoester compound is one selected from the group consisting of dimethoxymethane, diethoxymethane, 1,1-dimethoxyethane, 1,1-diethoxyethane, 1, 1-dimethoxypropane, 1,1-diethoxypropane, 2,2-dimethoxypropane, 2,2-diethoxypropane, 1,1-dimethoxybutane, 1,1-diethoxybutane, 2,2-dimethoxybutane, 2,2-diethoxybutane, orthomethyl formate, orthoethyl formate, orthomethyl acetate, and orthoethyl acetate.

5. The method as set forth in claim 1 or 2, wherein:
the compound represented by General Formula (2) is a ketone, an aldehyde, or an ester.

6. The method as set forth in claim 1 or 2, wherein:
the compound represented by General Formula (2) and the alcohol represented by General Formula (3) have boiling points of 200°C or lower.

7. The method as set forth in claim 1 or 2, wherein:
the cation portion is a quaternary ammonium cation or a cation having a heterocyclic skeleton having a nitrogen atom,
the quaternary ammonium cation being represented by General Formula (4): 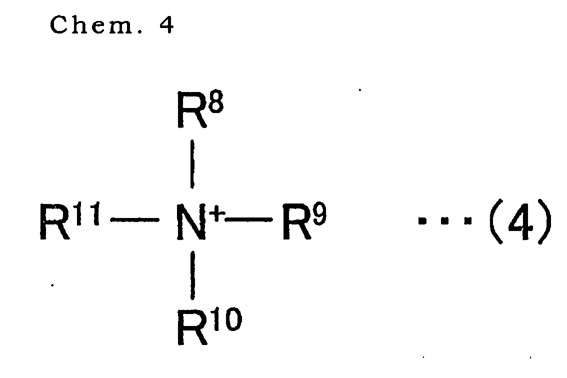 where R⁸ to R¹¹ are a saturated alkyl group(s), an unsaturated alkyl group(s), a cycloalkyl group(s), a heterocyclic group(s), an aryl group(s), or an alkoxyalkyl group(s).

8. The method as set forth in claim 7, wherein:
the cation having the heterocyclic skeleton is an imidazolium cation, a pyridinium cation or a triazine derivative cation,
the imidazolium cation being represented by General Formula (5) : 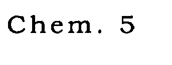 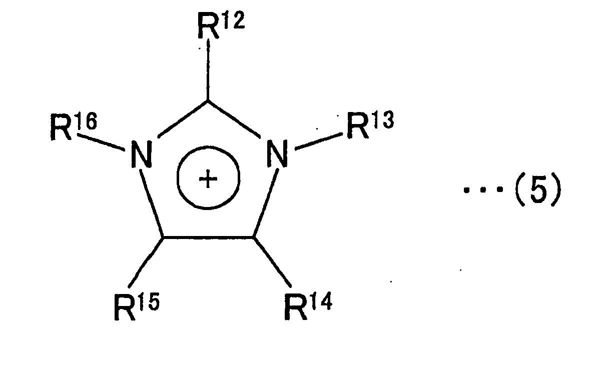 where R¹² to R¹⁶ are a hydrogen atom(s), a saturated alkyl group(s), an unsaturated alkyl group(s), a cycloalkyl group(s), an aryl group(s), or alkoxyalkyl group(s),
the pyridinium cation being represented by General Formula (6): 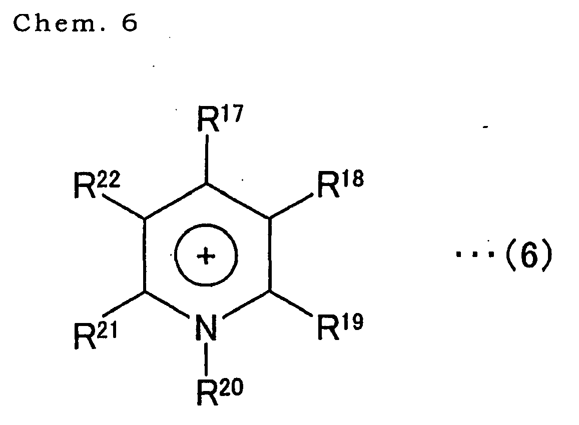 where R¹⁷ to R²² are a hydrogen atom(s), a saturated alkyl group(s), an unsaturated alkyl group(s), a cycloalkyl group(s), an aryl group(s), or an alkoxyalkyl group(s), and
the triazine derivative cation being represented by General Formula (7): 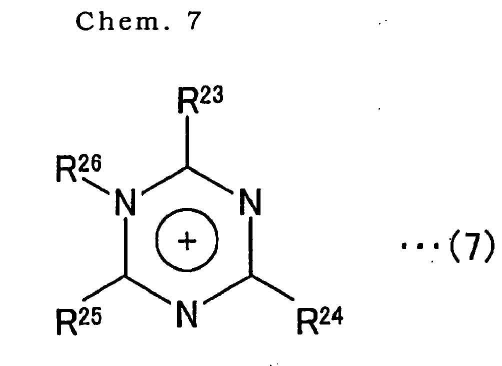 where R²³ to R²⁶ are a hydrogen atom(s), a saturated alkyl group(s), an unsaturated alkyl group(s), a cycloalkyl group(s), an aryl group(s), or an alkoxyalkyl group(s).

9. The method as set forth in claim 1 or 2, wherein:
the cation portion is a quaternary phosphonium cation, or a cation having a heterocyclic skeleton having a phosphorous atom,
the quaternary phosphonium cation being represented by General Formula (8): 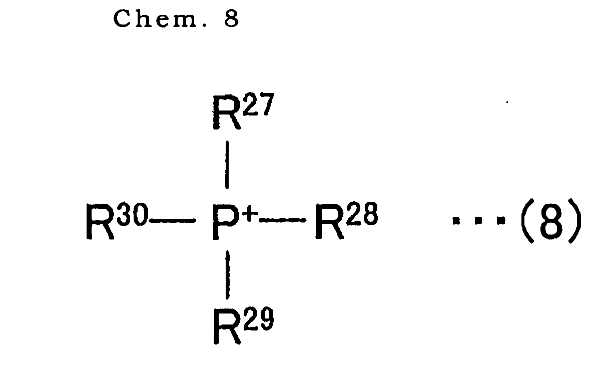 where R²⁷ to R³⁰ are a saturated alkyl group(s), an unsaturated alkyl group(s), a cycloalkyl group(s), a hetrocyclic group(s), an aryl group(s), a vinyl group(s), or an alkoxyalkyl group(s).

10. The method as set forth in claim 1 or 2, wherein:
the anion portion is one selected from the group consisting of: a halide ion, triflate anion, tetrafluoroborate anion, hexafluorophosphate anion, nitrate ion, sulfate ion, N(CF₃SO₂)₂-, N(CN)₂-, C(CN)₃-, CH₃OSO₃-, (C₂H₅)₂PO₄-, N(C₂F₅SO₂)₂-, N(CF₃CO)₂-, N(CF₃SO₂)(CF₃CO)-, N(FSO₂)₂-, and CF₃COO-.

## Patentansprüche

1. Verfahren zur Herstellung einer ionischen Flüssigkeit aus einer Salzschmelzenlösung, die eine Salzschmelze enthält, die durch Mischen des Kation-Anteils und des Anion-Anteils erzeugt wird, wobei die ionische Flüssigkeit die Salzschmelze als Hauptbestandteil enthält, welches Verfahren umfasst:
Entfernen von Wasser aus der Salzschmelzenlösung, wobei der Schritt des Entfernens des Wassers ein Hinzufügen einer Verbindung zu der Salzschmelzenlösung umfasst, wobei die Verbindung durch die allgemeine Formel (1) dargestellt ist: 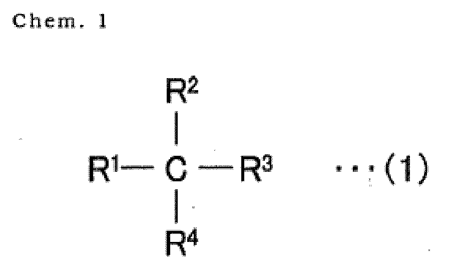 wobei zumindest zwei der Gruppen R¹ bis R⁴ C1- bis C8-Alkoxylgruppe(n) sind, wobei der Rest der Gruppen R¹ bis R⁴ (ein) Wasserstoffatom(e) oder (eine) C1- bis C8-Alkoxylgruppe(n) ist/sind, und
Reinigen einer Mischung, die durch den Schritt des Entfernens des Wassers erhalten wird, wobei der Schritt des Reinigens zumindest eine Verbindung, die durch eine allgemeine Formel (2) dargestellt ist, und einen Alkohol entfernt, der durch eine allgemeine Formel (3) dargestellt ist, wobei die allgemeine Formel (2) wie folgt lautet: 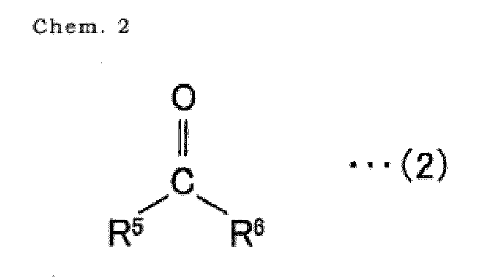 wobei R⁵ und R⁶ (ein) Wasserstoffatom(e), (eine) C1- bis C8-Alkylgruppe(n) oder (eine) C1- bis C8-Alkoxylgruppe(n) ist/sind, und
wobei die allgemeine Formel (3) wie folgt lautet:
Chem. 3
**R⁷-OH-** (3)
wobei R⁷ eine C1- bis C8-Alkylgruppe ist.

2. Verfahren nach Anspruch 1, weiterhin umfassend:
Synthetisieren der Salzschmelze,
wobei:
in dem Schritt des Entfernens des Wassers die Verbindung zu der Salzschmelzenlösung hinzugefügt wird, die in dem Schritt des Synthetisierens der Salzschmelze erhalten wird.

3. Verfahren nach Anspruch 1 oder 2, wobei:
die Verbindung, die durch die allgemeine Formel (1) dargestellt ist, eine Acetal- oder Orthoester-Verbindung ist.

4. Verfahren nach Anspruch 3, wobei:
die Acetal- oder Orthoester-Verbindung aus der Gruppe ausgewählt ist, die aus Dimethoxymethan, Diethoxymethan, 1,1-Dimethoxyethan, 1,1-Diethoxyethan, 1, 1-Dimethoxypropan, 1,1-Diethoxypropan, 2,2-Dimethoxypropan, 2,2-Diethoxypropan, 1,1-Dimethoxybutan, 1,1-Diethoxybutan, 2,2-Dimethoxybutan, 2,2-Diethoxybutan, Orthomethylformiat, Orthoethylformiat, Orthomethylacetat und Orthoethylacetat besteht.

5. Verfahren nach Anspruch 1 oder 2, wobei:
die Verbindung, die durch die allgemeine Formel (2) dargestellt ist, ein Aldehyd oder ein Ester ist.

6. Verfahren nach Anspruch 1 oder 2, wobei:
die Verbindung, die durch die allgemeine Formel (2) dargestellt ist, und der Alkohol, der durch die allgemeine Formel (3) dargestellt ist, Siedepunkte von 200°C oder niedriger haben.

7. Verfahren nach Anspruch 1 oder 2, wobei:
der Kation-Anteil ein quaternäres Ammonium-Kation oder ein Kation ist, das ein heterocyclisches Skelett mit einem Stickstoffatom aufweist,
wobei das quaternäre Ammonium-Kation durch die allgemeine Formel (4) dargestellt ist: 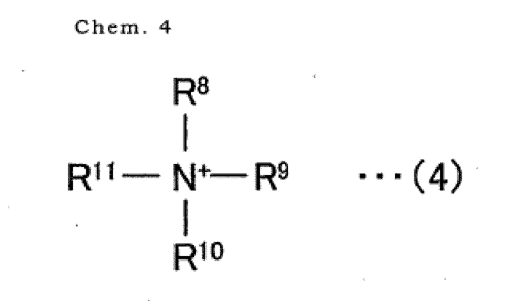 wobei R⁸ bis R¹¹ (eine) gesättigte Alkylgruppe(n), (eine) ungesättigte Alkylgruppe(n), (eine) Cycloalkylgruppe(n), (eine) heterocyclische Alkylgruppe(n), (eine) Arylgruppe(n) oder (eine) Alkoxyalkylgruppe(n) ist/sind.

8. Verfahren nach Anspruch 7, wobei:
das Kation, das das heterocyclische Skelett aufweist, ein Imidazol-Kation, ein Pyridinium-Kation oder ein von Triazin abgeleitetes Kation ist,
wobei das Imidazol-Kation durch die allgemeine Formel (5) dargestellt ist: 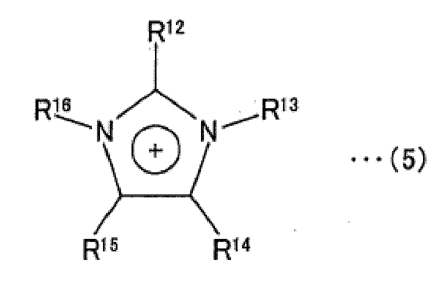 wobei R¹² bis R¹⁶ (ein) Wasserstofifatom(e), (eine) gesättigte Alkylgruppe(n), (eine) ungesättigte Alkylgruppe(n), (eine) Cycloalkylgruppe(n), (eine) Arylgruppe(n) oder (eine) Alkoxyalkylgruppe(n) ist/sind,
wobei das Pyridinium-Kation durch die allgemeine Formel (6) dargestellt ist: 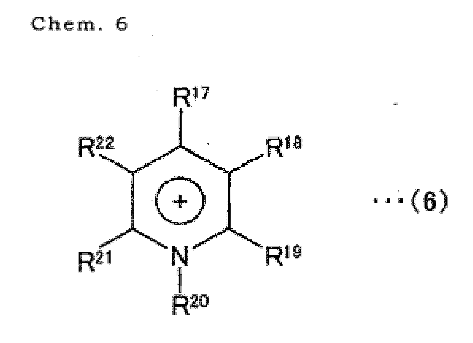 wobei R¹⁷ bis R²² (ein) Wasserstofifatom(e), (eine) gesättigte Alkylgruppe(n), (eine) ungesättigte Alkylgruppe(n), (eine) Cycloalkylgruppe(n), (eine) Arylgruppe(n) oder (eine) Alkoxyalkylgruppe(n) ist/sind, und
wobei das von Triazin abgeleitete Kation durch die allgemeine Formel (7) dargestellt ist: 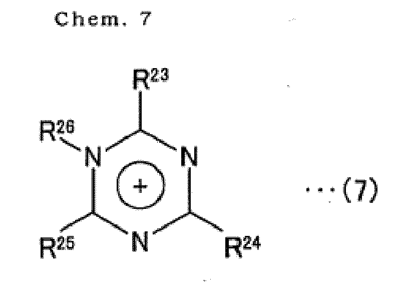 wobei R²³ bis R²⁶ (ein) Wasserstofifatom(e), (eine) gesättigte Alkylgruppe(n), (eine) ungesättigte Alkylgruppe(n), (eine) Cycloalkylgruppe(n), (eine) Arylgruppe(n) oder (eine) Alkoxyalkylgruppe(n) ist/sind.

9. Verfahren nach Anspruch 1 oder 2, wobei:
der Kation-Anteil ein quaternäres Phosphonium-Kation oder ein Kation ist, das ein heterocyclisches Skelett mit einem Phosphoratom aufweist,
wobei das quaternäre Phosphonium-Kation durch die allgemeine Formel (8) dargestellt ist: 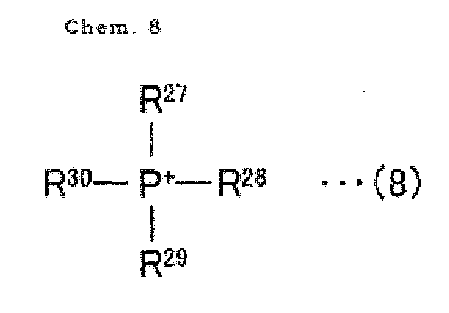 wobei R²⁷ bis R³⁰ (eine) gesättigte Alkylgruppe(n), (eine) ungesättigte Alkylgruppe(n), (eine) Cycloalkylgruppe(n), (eine) heterocyclische Alkylgruppe(n), (eine) Arylgruppe(n) oder (eine) Alkoxyalkylgruppe(n) ist/sind.

10. Verfahren nach Anspruch 1 oder 2, wobei:
der Anion-Anteil aus der Gruppe ausgewählt ist, die aus einem Halogenid-lon, einem Trifluoromethansulfonat-Anion, einem Tetrafluoroborat-Anion, einem Hexafluorophosphat-Anion, einem Nitrat-Ion, einem Sulfat-Ion, N(CF₃SO₂)₂-, N(CN)₂-, C(CN)₃-, CH₃OSO₃-, (C₂H₅)₂PO₄-, N(C₂F₅SO₂)₂-, N(CF₃CO)₂-, N(CF₃SO₂)(CF₃CO)-, N(FSO₂)₂- und CF₃COO- besteht.

## Revendications

1. Procédé de production d'un liquide ionique à partir d'une solution de sel fondu contenant un sel fondu constitué par la combinaison de la partie cations et de la partie anions, le liquide ionique contenant le sel fondu en tant que composant principal, le procédé comprenant:
élimination de l'eau de la solution de sel fondu, l'étape d'élimination de l'eau incluant l'addition d'un composé dans la solution de sel fondu, le composé étant représenté par la Formule Générale (1): 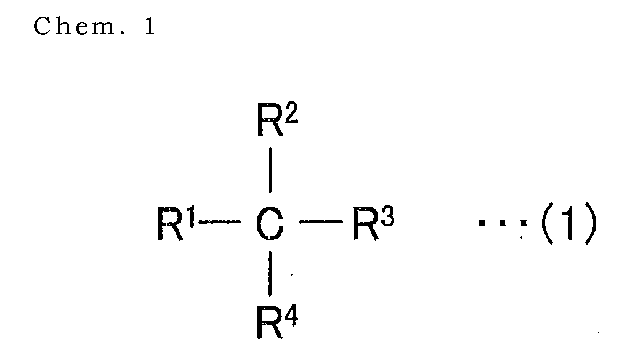 où au moins deux des radicaux R¹ à R⁴ sont un groupe(s) alcoxy en C1 à C8, le reste des radicaux R¹ à R⁴ sont un atome(s) d'hydrogène ou un groupe(s) alkyle en C1 à C8, et
purification d'un mélange obtenu par l'étape d'élimination de l'eau, l'étape de purification éliminant au moins un d'un composé représenté par la Formule Générale (2) et un alcool représenté par la Formule Générale (3),
la Formule Générale (2) étant comme suit: 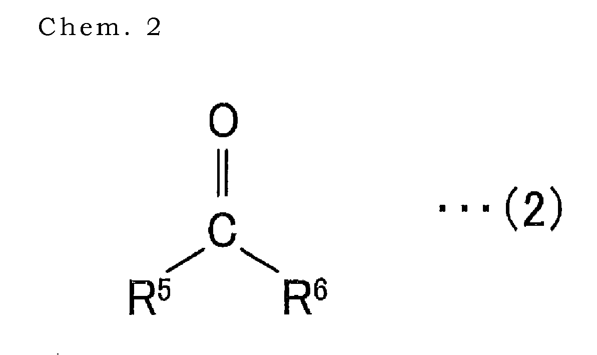 où R⁵ et R⁶ représentent un atome(s) d'hydrogène, un groupe(s) alkyle en C1 à C8, ou un groupe(s) alcoxy en C1 à C8, et
la Formule Générale (3) étant comme suit:
Chem. 3
R⁷-OH (3)
où R⁷ est un groupe alkyle en C1 à C8.

2. Procédé selon la revendication 1, comprenant en outre:
la synthèse du sel fondu,
dans lequel:
dans l'étape d'élimination de l'eau, le composé est ajouté à la solution de sel fondu obtenue dans l'étape de synthèse du sel fondu.

3. Procédé selon la revendication 1 ou 2, dans lequel:
le composé représenté par la Formule Générale (1) est un acétal ou un composé ortho-ester.

4. Procédé selon la revendication 3, dans lequel:
l'acétal ou le composé ortho-ester est choisi dans le groupe consistant en le diméthoxyméthane, le diéthoxyméthane, le 1,1-diméthoxyéthane, le 1,1-diéthoxyéthane, le 1,1-diméthoxypropane, 1,1-diéthoxypropane, le 2,2-diméthoxypropane, 2,2-diéthoxypropane, le 1,1-dimethoxybutane, le 1,1-diéthoxybutane, le 2,2-dimethoxybutane, le 2,2-diéthoxybutane, le formitate d'orthométhyle, le formiate d'orthoéthyle, l'acétate d'orthométhyle, et l'acétate d'orthoéthyle.

5. Procédé selon la revendication 1 ou 2, dans lequel:
le composé représenté par la Formule Générale (2) est une cétone, un aldéhyde, ou un ester.

6. Procédé selon la revendication 1 ou 2, dans lequel:
le composé représenté par la Formule Générale (2) et l'alcool représenté par la Formule Générale (3) ont des points d'ébullition de 200°C ou moins.

7. Procédé selon la revendication 1 ou 2, dans lequel:
la portion cation est un cation ammonium quaternaire ou un cation ayant un squelette hétérocyclique ayant un atome d'azote,
le cation d'ammonium quaternaire étant représenté par la Formule Générale (4): 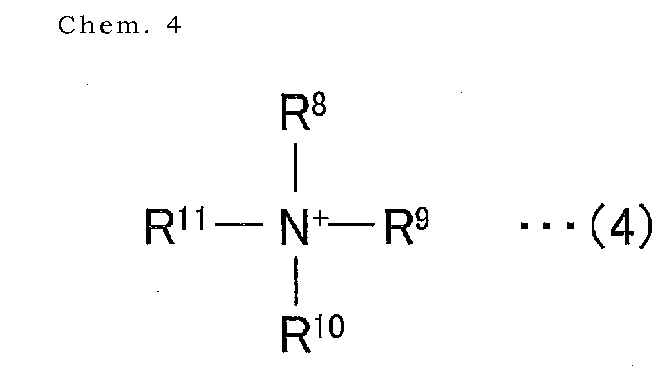 où R⁸ à R¹¹ sont un groupe(s) alkyle saturé, un groupe(s) alkyle insaturé, un groupe(s) cycloalkyle, un groupe(s) hétérocyclique, un groupe(s) aryle, ou un groupe(s) alcoxyalkyle.

8. Procédé selon la revendication 7, dans lequel:
le cation ayant le squelette hétérocyclique est un cation imidazolium, un cation pyridinium ou un cation dérivé de triazine,
le cation imidazolim étant représenté par la Formule Générale (5): 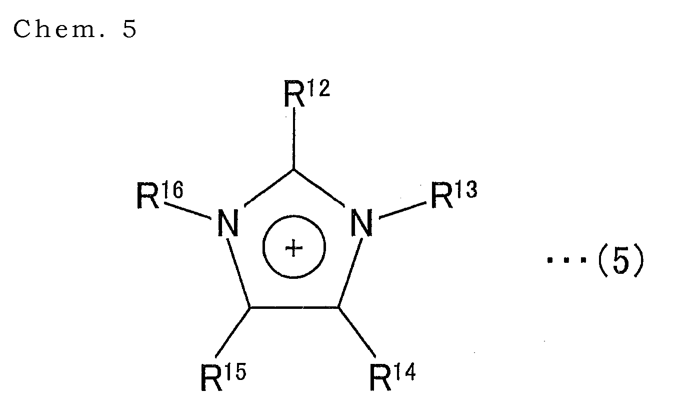 où R¹² à R¹⁶ sont un atome(s) d'hydrogène, un groupe(s) alkyle saturé, un groupe(s) alkyle insaturé, un groupe(s) cycloalkyle, un groupe(s) aryle ou un groupe(s) alkoxyalkyle,
le cation pyridinium étant représenté par la Formule Générale (6): 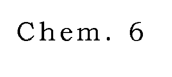 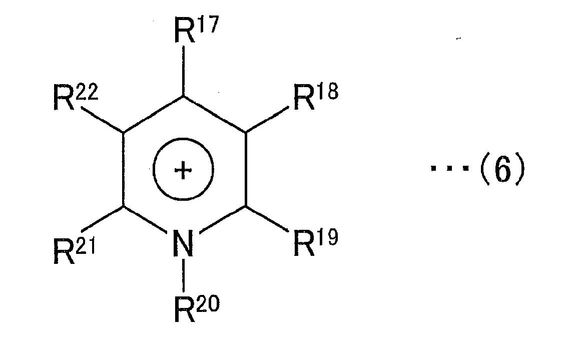 où R¹⁷ à R²² sont un atome(s) d'hydrogène, un groupe(s) alkyle saturé, un groupe(s) alkyle insaturé, un groupe(s) cycloalkyle, un groupe(s) aryle, ou un groupe(s) alcoxyalkyle, et
le cation dérivé de triazine étant représenté par la Formule Générale (7): 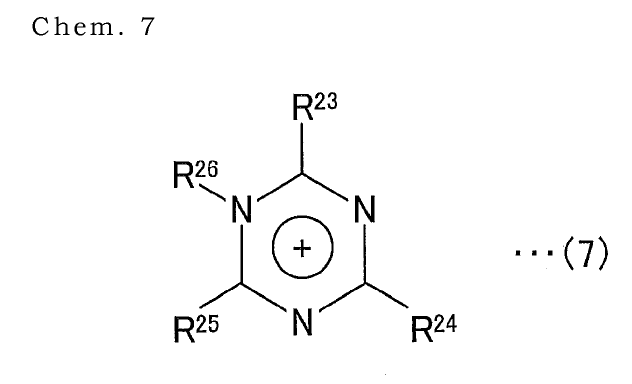 où R²³ à R²⁶ sont un atome(s) d'hydrogène, un groupe(s) alkyle saturé, un groupe(s) alkyle insaturé, un groupe(s) cycloalkyle, un groupe(s) aryle, ou un groupe(s) alcoxyalkyle.

9. Procédé selon la revendication 1 ou 2, dans lequel:
la portion cation est un cation phosphonium quaternaire ou un cation ayant un squelette hétérocyclique ayant un atome de phosphore,
le cation phosphonium quaternaire étant représenté par la formule générale (8): 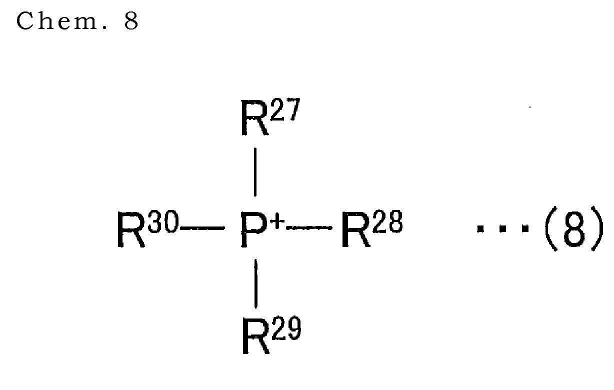 où R²⁷ à R³⁰ est un groupe(s) d'alkyle saturé, un groupe(s) alkyle insaturé, un groupe(s) cycloalkyle, un groupe(s) hétérocyclique, un groupe(s) aryle, un groupe(s) vinyle, ou un groupe(s) alkoxyalkyle.

10. Procédé selon la revendication 1 ou 2, dans lequel:
la partie anion est choisie dans le groupe consistant en: un ion halogénure, un anion triflate, un anion tétrafluoroborate, un anion hexafluorophosphate, un ion nitrate, un ion sulfate, N(CF₃SO₂)₂-, N(CN)₂-, C(CN)₃-, CH₃OSO₃-, (C₂H₅)₂PO₄-, N(C₂F₅SO₂)₂-, N(CF₃CO)₂-, N(CF₃SO₂)(CF₃CO)-, N(FSO₂)₂-, et CF₃COO-.
